(19) Europäisches Patentamt
European Patent Office
Office européen des brevets

(11) Veröffentlichungsnummer: **0 387 568 B1**

(12) # EUROPÄISCHE PATENTSCHRIFT

(45) Veröffentlichungstag der Patentschrift: **14.12.94**

(51) Int. Cl.$^{5}$: **C07D 471/04**, A01N 43/90, //(C07D471/04,221:00,221:00)

(21) Anmeldenummer: **90103619.4**

(22) Anmeldetag: **24.02.90**

(54) **Substituierte 1,8-Naphthyridine, Verfahren zu ihrer Herstellung und ihre Verwendung als Antidots.**

(30) Priorität: **11.03.89 DE 3907937**

(43) Veröffentlichungstag der Anmeldung:
**19.09.90 Patentblatt 90/38**

(45) Bekanntmachung des Hinweises auf die Patenterteilung:
**14.12.94 Patentblatt 94/50**

(84) Benannte Vertragsstaaten:
**BE CH DE DK ES FR GB IT LI NL**

(56) Entgegenhaltungen:
**EP-A- 0 329 012**
**DE-A- 3 601 688**
**DE-A- 3 644 825**

**W. THEILHEIMER, Repertorium 4, 1950, Basel-New York S. KARGER "Synthetische Methoden der Oranischen Chemie " Seite 260, n 732 * Naphthyridine ***

**J. Chem. Soc. (c) 1967, Seiten 1564-8**

(73) Patentinhaber: **BASF Aktiengesellschaft**
**Carl-Bosch-Strasse 38**
**D-67063 Ludwigshafen (DE)**

(72) Erfinder: **Saupe, Thomas, Dr.**
**Kressenwiesenweg 13**
**D-6902 Sandhausen (DE)**
Erfinder: **Schaefer, Peter, Dr.**
**Paray-Le-Monial-Strasse 2**
**D-6702 Bad Duerkheim (DE)**
Erfinder: **Meyer, Norbert, Dr.**
**Dossenheimer Weg 22**
**D-6802 Ladenburg (DE)**
Erfinder: **Wuerzer, Thomas, Dr.**
**Ruedigerstrasse 13**
**D-6701 Otterstadt (DE)**
Erfinder: **Westphalen, Karl-Otto, Dr.**
**Mausbergweg 58**
**D-6720 Speyer (DE)**

**EP 0 387 568 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft substituierte 1,8-Naphthyridine der allgemeinen Formel I

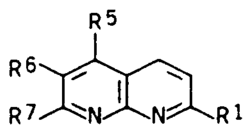

I,

in der die Substituenten die folgende Bedeutung haben:

- $R^1$ Mercapto, Benzylthio, Sulfonyl, Amino oder $NR^2R^3$, wobei
- $R^2$ $C_1$-$C_{12}$-Alkyl, $C_3$-$C_6$-Alkenyl, $C_3$-$C_8$-Cycloalkyl, Phenyl, Phenyl-($C_1$-$C_3$)-alkyl, ein- bis dreifach durch Halogen, $C_1$-$C_4$-Alkyl, $C_1$-$C_4$-Alkoxi, Amino, Mono- oder Dialkylamino mit 1 bis 6 C-Atomen je Alkylrest substituiertes Phenyl oder Phenyl-($C_1$-$C_3$)-alkyl oder eine Gruppe

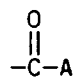

,

worin A für $C_1$-$C_8$-Alkyl, Phenyl, ein- bis dreifach durch Halogen, $C_1$-$C_4$-Alkyl, $C_1$-$C_4$-Alkoxy, Amino, Mono- oder Dialkylamino mit 1 bis 6 C-Atomen je Alkylrest substituiertes Phenyl, Amino, Alkyl- oder Dialkylamino mit jeweils 1 bis 6 C-Atomen je Alkylrest, Phenylamino, Morpholino oder Piperidyl steht, und
- $R^3$ Wasserstoff, $C_1$-$C_{12}$-Alkyl, $C_3$-$C_8$-Cycloalkyl bedeuten oder
- $R^2$ und $R^3$ miteinander zu einem gesättigten oder ungesättigten 5- oder 6-gliedrigen Heterocyclus, der zusätzlich zu dem Stickstoffatom, an sie gebunden sind ein weiteres Heteroatom wie Sauerstoff oder Stickstoff enthalten kann, verbunden sind;

Hydroxyl, Mercapto, Halogen oder
- $XR^4$, worin X Sauerstoff oder Schwefel bedeutet und $R^4$ für $C_1$-$C_{12}$-Alkyl, $C_1$-$C_4$-Alkoxicarbonyl-($C_1$-$C_4$)-alkyl, $C_5$-$C_8$-Cycloalkyl, Phenyl oder Phenyl-$C_1$-$C_3$-alkyl, wobei die aromatischen Ringe ein bis drei der folgenden Reste tragen können: Halogen, $C_1$-$C_4$-Alkyl, $C_1$-$C_4$-Alkoxi, Amino, Mono- oder Dialkylamino mit 1 bis 6 C-Atomen je Alkylrest,
- $R^5$, Wasserstoff, $C_1$-$C_{12}$-Alkyl, $C_3$-$C_8$-Cycloalkyl,
- $R^7$, Phenyl oder Phenyl-($C_1$-$C_3$)-alkyl, wobei der Phenylkern jeweils seinerseits ein bis drei der folgenden Gruppen tragen kann: Halogen, $C_1$-$C_4$-Alkyl, $C_1$-$C_4$-Alkoxy, $C_1$-$C_4$-Halogenalkyl, Amino oder Mono- oder Dialkylamino mit 1 bis 4 C-Atomen je Alkylrest; Hydroxy, Halogen, Mercapto, $C_1$-$C_4$-Alkoxy, $C_1$-$C_4$-Alkylthio, Amino, Mono- oder Dialkylamino mit 1 bis 6 C-Atomen je Alkylrest, Phenoxy oder Phenylthio, wobei der Phenylkern jeweils seinerseits ein bis drei der folgenden Gruppen tragen kann: Halogen, $C_1$-$C_4$-Alkyl, $C_1$-$C_4$-Alkoxy, $C_1$-$C_4$-Halogenalkyl, Amino oder Mono- oder Dialkylamino mit 1 bis 4 C-Atomen je Alkylrest; Halogen-($C_1$-$C_3$)-alkyl, $C_1$-$C_4$-Alkoxy-($C_1$-$C_3$)-alkyl, $C_1$-$C_4$-Alkylthio-($C_1$-$C_3$)-alkyl, Amino-($C_1$-$C_3$)-alkyl, $C_1$-$C_4$-Acyloxy, Hydrazino, welches ein bis drei $C_1$-$C_4$-Alkylreste und/oder einen $C_1$-$C_4$-Acylrest tragen kann; Cyano und Thiocyanato,
- $R^6$ Wasserstoff, $C_1$-$C_{12}$-Alkyl, $C_3$-$C_8$-Cycloalkyl, Phenyl oder Phenyl-($C_1$-$C_3$)-alkyl, wobei der Phenylkern jeweils seinerseits ein bis drei der folgenden Gruppen tragen kann: Halogen, $C_1$-$C_4$-Alkyl, $C_1$-$C_4$-Alkoxy, $C_1$-$C_4$-Halogenalkyl, Amino oder Mono- oder Dialkylamino mit 1 bis 4 C-Atomen je Alkylrest; Halogen, Halogen-($C_1$-$C_3$)-alkyl, $C_1$-$C_4$-Alkoxy-($C_1$-$C_3$)-alkyl, $C_1$-$C_4$-Alkylthio-($C_1$-$C_3$)-alkyl, Amino-($C_1$-$C_3$)-alkyl, Carboxyl, $C_1$-$C_4$-Alkoxycarbonyl,

wobei $R^1$ nicht Amino bedeutet, wenn $R^5$ und $R^6$ für Wasserstoff und $R^7$ für Wasserstoff oder Phenyl steht, sowie deren pflanzenverträgliche Salze.

Außerdem betrifft die Erfindung Verfahren zur Herstellung der Verbindungen I sowie herbizide Mittel, die 2-(4-Heteroaryloxy)- oder 2-(4-Aryloxy)phenoxyessig- oder -propionsäurederivate und/oder Cyclohexenonderivate als herbizide Wirkstoffe und substituierte 1,8-Naphthyridine als Antidots enthalten, sowie Verfahren zur selektiven Bekämpfung von unerwünschtem Pflanzenwuchs mit diesen herbiziden Mitteln.

Eine Übersicht über die Herstellung von verschiedenen 1,8-Naphthyridin-Derivaten findet sich in dem Beitrag von P.A.Lowe in "Comprehensive Heterocyclic Chemistry" (editors: A.R.Katritzky und C.W.Rees)

Vol. 2, 1st ed. (1984), Kap. 2.11. Weiterhin wird die Synthese von 1,8-Naphthyridin-Derivaten von P.Caluwe in Tetrahedron Bd. 36, Seiten 2359-2407, insbesondere Seite 2391-2394 (1980) beschrieben.

Eine pflanzenschützende Wirkung dieser Verbindungsklasse ist dem Stand der Technik nicht zu entnehmen.

Herbizide Wirkstoffe aus der Gruppe der 2-(4-Heteroaryloxy)- oder 2-(4-Aryloxy)-phenoxyessigsäurederivate der Formel IV,

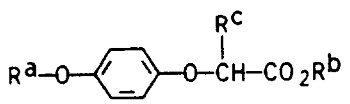

IV

in der die Substituenten folgende Bedeutung haben:

- $R^a$ ein Phenylring, ein Pyridylring, ein Benzoxazylrest, ein Benzthiazylrest oder ein Benzpyrazinylrest, wobei diese aromatischen Ringsysteme bis zu zwei der folgenden Reste tragen können: Halogen, Nitro, $C_1$-$C_4$-Alkyl, $C_1$-$C_4$-Halogenalkyl und/oder $C_1$-$C_4$-Halogenalkoxy,
- $R^b$ Wasserstoff, eine $C_1$- $C_4$-Alkylgruppe oder das Äquivalent eines pflanzenverträglichen Kations und
- $R^c$ Wasserstoff oder eine Methylgruppe,

sind aus der Literatur, z.B. aus DE-A-22 23 894, DE-A-24 33 067, DE-A-25 76 251, DE-A-30 04 770, BE-A-868 875 und BE-A-858 618 bekannt.

Sie dienen der Bekämpfung von unerwünschten Pflanzen aus der Familie der Gramineen. Die Verträglichkeit dieser Substanzen für Kulturpflanzen variiert jedoch zwischen kommerziell akzeptabel und unverträglich, je nach Substituenten und Aufwandmenge.

Ebenso verhält es sich mit Cyclohexenonderivaten der Formel V,

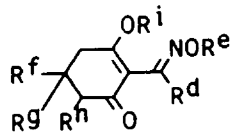

V

in welcher die Substituenten folgende Bedeutung haben:

- $R^d$ eine $C_1$-$C_4$-Alkylgruppe;
- $R^e$ eine $C_1$-$C_4$-Alkylgruppe, eine $C_3$-$C_4$-Alkenylgruppe, eine $C_3$-$C_4$-Alkinylgruppe, eine $C_3$-$C_4$-Halogenalkylengruppe oder eine Thenylgruppe, welche durch ein Halogenatom substituiert sein kann;
- $R^f$ eine $C_1$-$C_4$-Alkylgruppe, welche ein- oder zweifach durch $C_1$-$C_4$-Alkylthio oder $C_1$-$C_4$-Alkoxy substituiert sein kann; ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, und dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;
  ein 10-gliedriger gesättigter oder einfach ungesättigter Heterocyclus, welcher zwei Sauerstoffatome oder Schwefelatome enthält und durch bis zu drei $C_1$-$C_4$-Alkylgruppen und/oder Methoxygruppen substituiert sein kann;
  ein Phenylrest, ein Pyridylrest ein Thiazolylrest, ein Pyrazolylrest, ein Pyrrolylrest oder ein Isoxazolylrest, wobei diese Reste bis zu drei der folgenden Gruppen tragen können: $C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und/oder Benzoylamino
- $R^g$ Wasserstoff, Hydroxy oder, wenn $R^f$ die Bedeutung $C_1$-$C_6$-Alkylgruppe hat, eine $C_1$-$C_6$-Alkylgruppe;
- $R^h$ Wasserstoff, eine Cyanogruppe, ein Halogenatom, eine $C_1$-$C_4$-Alkoxycarbonylgruppe oder eine $C_1$-$C_4$-Alkylketoximgruppe und
- $R^i$ Wasserstoff oder das Äquivalent eines umweltverträglichen Kations.

Sie sind in der Literatur (z.B. EP-A 228 598, EP-A 230 235, EP-A 238 021, US-A 4 432 786, DE-A 24 39 104) ebenfalls als Herbizide beschrieben und dienen vorwiegend zur Bekämpfung unerwünschter Gräser in dicotylen Kulturen und in Gräsern, die nicht zur Familie der Gramineen zählen. Je nach Struktur der Substituenten und Dosis der Anwendung können Verbindungen aus dieser Gruppe auch zur selektiven Bekämpfung von unerwünschten Gräsern in Gramineen Kulturen wie Weizen und Reis eingesetzt werden.

Außerdem sind die Verbindungen 2-Amino-1,8-naphthyridin und 2-Amino-7-phenyl-1,8-naphthyridin in der Literatur beschrieben (J. Chem. Soc., (c) 1564 (1967)).

Der vorliegenden Erfindung lag nun die Aufgabe zugrunde, Verbindungen zu finden, welche die Nachteile, die bei der Verwendung der obengenannten Herbizide der Formeln IV und V bestehen, zumindest so stark zu verringern, daß der Ernteertrag der Kulturpflanzen nicht mehr oder nicht nennenswert herabgesetzt wird.

Entsprechend der Aufgabe wurden die eingangs definierten substituierten 1,8-Naphthyridine I gefunden. Weiterhin wurden Verfahren zur Herstellung dieser Verbindungen I sowie zur gemeinsamen Anwendung dieser Verbindungen mit den Herbiziden IV und V zur Beeinflussung unerwünschten Pflanzenwachstums gefunden. Weiterhin betrifft die Erfindung Mittel, welche die Verbindungen I enthalten, wobei es unerheblich ist, ob der herbizide Wirkstoff und die antidotische Verbindung gemeinsam oder getrennt formuliert und ausgebracht werden bzw. bei getrennter Ausbringung, in welcher Reihenfolge herbizider Wirkstoff und Antidot appliziert werden.

Die erfindungsgemäßen Verbindungen I sind ausgehend von 2,6-Diaminopyridin II durch Umsetzung mit einer $\beta$-Dicarbonylverbindung III zugänglich.

Das Substitutionsmuster kann durch entsprechende Wahl der $\beta$-Dicarbonylverbindung III in einem weiten Bereich variiert werden; neben 1,3-Diketonen, Malonaldehyden bzw. ihren Acetalen und $\beta$-Ketoaldehyden bzw. ihren Acetalen können auch Malonester, $\beta$-Ketosäureester und $\beta$-Formylsäureester bzw. ihre Acetale für die Cyclokondensation zu I eingesetzt werden.

Die Herstellung von I sei anhand folgender Reaktionsgleichungen demonstriert:

a)

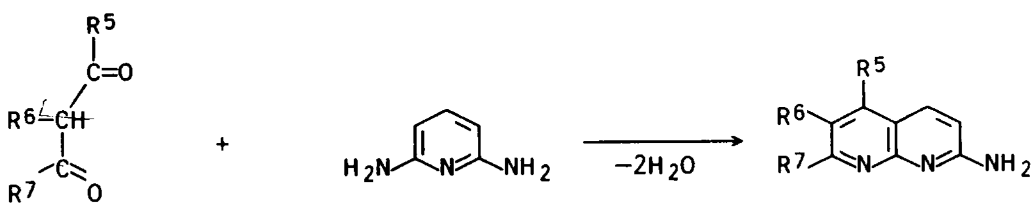


III II I

z.B. mit

$R^5$, $R^7$ = H, Alkyl, Cycloalkyl, Phenyl, Phenylalkyl, Halogenalkyl, Alkoxyalkyl sowie

$R^6$ = H, Alkyl, Cycloalkyl, Phenyl, Phenylalkyl, Halogen, Halogenalkyl, Alkoxyalkyl

b)

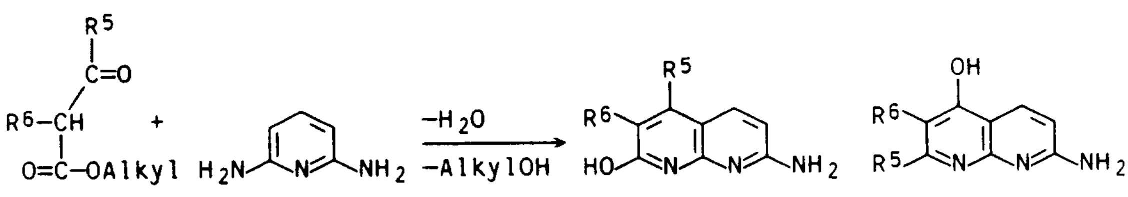


III II Ia bzw. Ib

Die strukturisomeren Verbindungen Ia bzw. Ib entstehen im Gemisch, können aber durch übliche Trennverfahren, z.B. durch Umkristallisation oder Chromatographie einzeln erhalten werden.

c)

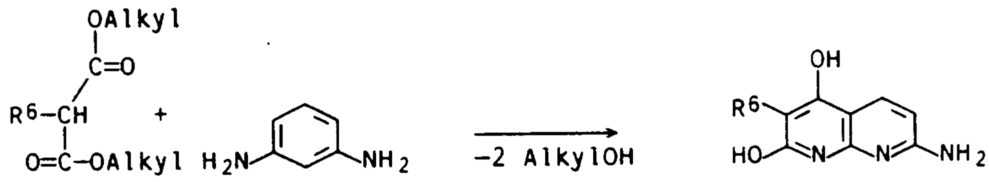

III II Ic

2- bzw. 4-Hydroxy-1,8-naphthyridine Ia bzw. Ib sowie 2,4-Dihydroxy-1,8-naphthyridine Ic reagieren mit einem Halogenierungsmittel wie Phosphoroxychlorid oder -bromid zu 2- bzw. 4-Halogennaphthyridinen, respektive 2,4-Dihalogennaphthyridinen. Durch Austauschreaktionen des Halogens gegen H-, S- oder O-Nukleophile sowie C-Nukleophile wie Cyanid kann das Substitutionsmuster von I in einem weiten Bereich variiert werden.

Weiterhin kann die primäre Amino-Funktion der substituierten 1,8-Naphthyridine I in an sich bekannter Weise derivatisiert, wie z.B. alkyliert oder acyliert, werden. Auch ist es möglich, die primäre Amino-Gruppe von I durch Diazotierung in saurer wäßriger Lösung und Verkochung des resultierenden Diazoniumsalzes in eine Hydroxy-Gruppe zu überführen.

Die so dargestellten substituierten 1,8-Naphthyridine I mit $R^1$ = OH reagieren analog wie oben beschrieben mit Halogenierungsmitteln wie Phosphoroxychlorid oder -bromid zu den entsprechenden 1,8-Naphthyridinen I mit $R^1$ = Halogen.

Das auf diese Weise anstelle der primären Amino-Funktion von I eingeführte Halogen ist den bereits oben genannten nukleophilen Austauschreaktionen zugänglich, so daß weitere Variationen des Substitutionsmusters von I möglich sind.

Üblicherweise setzt man die Ausgangsstoffe II und III in stöchiometrischem Verhältnis ein. Ein überschuß des einen oder anderen kann in Einzelfällen aber durchaus vorteilhaft sein.

Die Umsetzung kann kontinuierlich oder diskontinuierlich bei Normal-, über- oder Unterdruck nach den dafür üblichen Techniken durchgeführt werden. Die Reaktionstemperatur liegt im allgemeinen bei 20 bis 400, insbesondere bei 50 bis 300 °C, vorteilhaft im Siedebereich des Lösungsmittels.

Als Lösungs- und Kondensationsmittel dienen vorteilhafterweise Säuren wie Schwefelsäure, Phosphorsäure, Essigsäure und entsprechende Gemische sowie höhersiedende Ether wie Diphenylether, Dioxan oder Tetrahydrofuran. In bestimmten Fällen kann es auch von Vorteil sein, die beiden Ausgangsverbindungen I und II ohne Lösungsmittel, also in Substanz, zu erhitzen.

Neben den bereits genannten kommen aber auch Lösungsmittel wie aliphatische und aromatische, gegebenenfalls chlorierte Kohlenwasserstoffe in Frage.

Im Hinblick auf die bestimmungsgemäße Verwendung der Verbindungen I als pflanzenschützende Mittel kommen als Substituenten folgende Reste in Betracht:

$R^1$ Mercapto, Benzylthio, Hydroxy, $C_1$-$C_4$-Alkoxy, Sulfonyl ($SO_3H$), Amino $NR^2R^3$, wobei

$R^2$ $C_1$-$C_{12}$-Alkyl, $C_3$-$C_6$-Alkenyl, $C_3$-$C_8$-Cycloalkyl, Phenyl, Phenyl-($C_1$-$C_3$)-alkyl, durch Halogen, $C_1$-$C_4$-Alkyl, $C_1$-$C_4$-Alkoxi, Amino, Mono- oder Dialkylamino mit 1 bis 6 C-Atomen je Alkylrest substituiertes Phenyl oder Phenyl($C_1$-$C_3$)-alkyl oder eine Gruppe

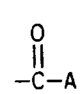 ,

worin A für $C_1$-$C_8$-Alkyl, Phenyl, ein- bis dreifach durch Halogen, $C_1$-$C_4$-Alkyl, $C_1$-$C_4$-Alkoxy, Amino, Mono- oder Dialkylamino mit 1 bis 6 C-Atomen je Alkylrest substituiertes Phenyl, Amino, Alkyl- oder Dialkylamino mit jeweils 1 bis 6 C-Atomen je Alkylrest substituiertes Phenyl, Phenylamino, Morpholino oder Piperidyl steht, und

$R^3$ Wasserstoff, $C_1$-$C_{12}$-Alkyl, $C_3$-$C_8$-Cycloalkyl bedeuten oder

$R^2$ und $R^3$ miteinander zu einem gesättigten oder ungesättigten 5- oder 6-gliedrigen Heterocyclus, der ein weiteres Heteroatom wie Sauerstoff oder Stickstoff enthalten kann, verbunden sind; Hydroxyl, Mercapto, Halogen, insbesondere Fluor, Chlor und Brom oder

$XR^4$, worin X Sauerstoff oder Schwefel bedeutet und $R^4$ für $C_1$-$C_{12}$-Alkyl, $C_1$-$C_4$-Alkoxicarbonyl-($C_1$-$C_4$)-alkyl, $C_5$-$C_8$-Cycloalkyl, Phenyl, Phenyl substituiert durch Halogen, $C_1$-$C_4$-Alkyl, $C_1$-$C_4$-Alkoxi, Amino, Mono- oder Dialkylamino mit 1 bis 6 C-Atomen je Alkylrest, ggf. substituiertes Phenyl-($C_1$-$C_3$)-alkyl,

$R^5$, Wasserstoff,

$R^7$ $C_1$-$C_{12}$-Alkyl, insbesondere $C_1$-$C_8$-, besonders bevorzugt $C_1$-$C_4$-Alkyl wie bei $R^2$ genannt,
$C_3$-$C_8$-Cycloalkyl, insbesondere Cyclopropyl, Cyclopentyl und Cyclohexyl,
Phenyl oder Phenyl-($C_1$-$C_3$)-alkyl, wobei der Phenylkern jeweils gegebenenfalls substituiert ist durch $C_1$-$C_4$-Alkyl, Halogen wie Fluor, Chlor oder Brom, $C_1$-$C_4$-Alkoxy wie Methoxy, Ethoxy, Propoxy,
Isopropoxy, Butoxy, tert.-Butoxy, $C_1$-$C_4$-Halogenalkyl, z.B. $CF_3$, Amino, Mono- oder Dialkylamino mit 1 bis 4-C-Atomen je Alkylrest, als Phenylalkylrest kommt insbesondere gegebenenfalls substituiertes Benzyl- oder Phenylethyl in Betracht, Hydroxy, Mercapto, Halogen wie Chlor oder Brom $C_1$-$C_4$-Alkoxy oder Alkylthio wie Methoxy, Ethoxy, Propoxy, Isopropoxy, Butoxy, tert. -Butoxy oder die entsprechenden Alkylthioreste, Amino, Mono- oder Dialkylamino mit 1 bis 6 C-Atomen je Alkylrest z.B. wie für $R^2$ genannt,
Phenoxy- oder Phenylthio, wobei der Phenylkern jeweils gegebenenfalls die für Phenyl bzw. Phenyl-($C_1$-$C_3$)-alkyl genannten Substituenten tragen kann,
Halogen-($C_1$-$C_3$)-alkyl wie z.B. Trifluormethyl, Trichlormethyl, 1,2-Difluorethyl,
$C_1$-$C_4$-Alkoxy- oder -Alkylthio-($C_1$-$C_3$)alkyl wie z.B. Methoxymethyl oder Methylmercaptomethyl,
Amino-($C_1$-$C_3$)-alkyl wie z.B. Dimethylaminomethyl,
$C_1$-$C_4$-Acyloxy
Hydrazino, das gegebenenfalls substituiert ist durch ein bis drei $C_1$-$C_4$-Alkylreste, insbesondere durch zwei geminal oder vicinal angeordnete $C_1$-$C_4$-Alkylreste wie z.B. Methyl oder Ethyl oder monoacyliert ist, wobei als Acylrest $C_1$-$C_4$-Acyl, insbesondere Acetyl in Betracht kommt,
Cyano, Thiocyanato,

$R^6$ Wasserstoff,
$C_1$-$C_{12}$-Alkyl, insbesondere $C_1$-$C_4$-Alkyl wie für $R^2$ genannt, $C_3$-$C_8$-Cycloalkyl, insbesondere Cyclopropyl, Cyclopentyl, Cyclohexyl, gegebenenfalls substituiertes Phenyl oder Phenyl-($C_1$-$C_3$)-alkyl wie für $R^5$, $R^7$ genannt,
Halogen oder Halogen-($C_1$-$C_3$)-alkyl jeweils wie für $R^5$, $R^7$ genannt, $C_1$-$C_4$-Alkoxy- oder -Alkylthio- oder Amino($C_1$-$C_3$)-alkyl jeweils wie für $R^5$, $R^7$ genannt,
Carboxyl,
$C_1$-$C_4$-Alkoxycarbonyl, z.B. Methoxy- oder Ethoxycarbonyl.

Als Salze der Verbindungen der Formel I kommen landwirtschaftlich brauchbare Salze, beispielsweise Alkalimetallsalze, insbesondere das Kalium- oder Natriumsalz, Erdalkalimetallsalze, insbesondere das Calcium-, Magnesium- oder Bariumsalz, Mangan-, Kupfer-, Zink- oder Eisensalze sowie Ammonium, Phosphonium-, Sulfonium- oder Sulfoxoniumsalze, beispielsweise Ammmoniumsalze, Tetraalkylammoniumsalze, Benzyltrialkylammoniumsalze, Trialkylsulfoniumsalze oder Trialkylsulfoxoniumsalze in Betracht.

Spezielle Beispiele für herbizide (Heteroaryloxy)- bzw. Aryloxy-phenoxyessigsäurederivate der Formel IV, deren Kulturpflanzenverträglichkeit durch substituierte 1,8-Naphthyridine der Formel I verbessert werden kann, sind in der folgenden Tabelle A aufgeführt.

Tabelle A

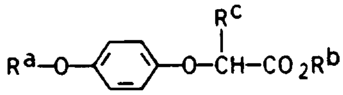

IV

| Nr. | $R^a$ | $R^b$ | $R^c$ | Lit |
|---|---|---|---|---|
| IV.1 | 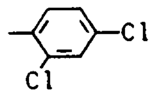 | $CH_3$ | $CH_3$ | DE-A 22 23 894 |
| IV.2 | 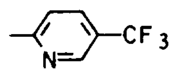 | $n-C_4H_9$ | $CH_3$ | BE-A 868 875 |
| IV.3 | 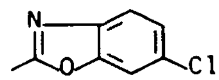 | $C_2H_5$ | $CH_3$ | BE-A 858 618 |
| IV.4 | 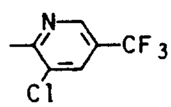 | $CH_3$ | $CH_3$ | BE-A 868 875 |
| IV.5 | 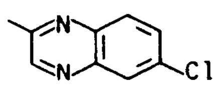 | $C_2H_5$ | $CH_3$ | DE-A 30 04 770 |

Spezielle Beispiele für Cyclohexenone der Formel V, deren Kulturpflanzenverträglichkeit durch substituierte 1,8-Naphthyridine der Formel I verbessert werden kann, sind in der folgenden Tabelle B aufgeführt.

Tabelle B

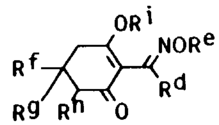

V

| Nr. | $R^d$ | $R^e$ | $R^f$ | $R^g$ | $R^h$ | $R^i$ | Literatur |
|---|---|---|---|---|---|---|---|
| V.1 | $C_3H_7$ | $CH_2CH=CH_2$ | $CH_3$ | $CH_3$ | $CO_2CH_3$ | Na | DE-A 2 439 104 |
| V.2 | $C_3H_7$ | $CH_2CH_3$ | $CH_2CH(CH_3)SCH_2CH_3$ | H | H | H | DE-A 2 822 304 |
| V.3 | $C_2H_5$ | $CH_2CH=CHCl$ | $CH_2CH(CH_3)SCH_2CH_3$ | H | H | H | US-A 4 440 566 |
| V.4 | $C_3H_7$ | $CH_2CH=CHCl$ | $CH_2CH(CH_3)SCH_2CH_3$ | H | H | H | US-A 4 440 566 |
| V.5 | $C_3H_7$ | $C_2H_5$ | 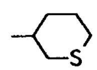 | H | H | H | EP-A 71 707 |
| V.6 | $C_2H_5$ | $C_2H_5$ | 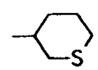 | H | H | H | EP-A 71 707 |
| V.7 | $CH_3$ | $CH_2CH=CHCH_3$ | 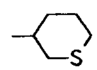 | H | H | H | EP-A 71 707 |
| V.8 | $C_3H_7$ | $C_2H_5$ | 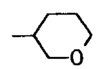 | H | H | H | EP-A 71 707 |
| V.9 | $C_2H_5$ | $CH_2CH=CHCl$ | 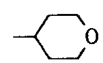 | H | H | H | EP-A 142 741 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.10 | $C_3H_7$ | $C_2H_5$ | 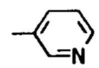 | H | H | H | EP-A 66 195 |
| V.11 | $C_2H_5$ | $C_2H_5$ | 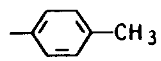 | H | H | H | DE-A 24 39 104 |
| V.12 | $C_2H_5$ | $CH_2CH=CHCH_3$ | 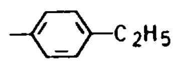 | H | H | H | DE-A 38 08 072 |
| V.13 | $C_2H_5$ | $C_2H_5$ | 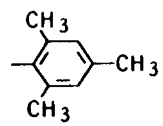 | H | H | H | EP-A 88 301 |
| V.14 | $C_3H_7$ | $CH_2CH=CHCl$ | 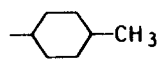 | H | H | H | EP-A 88 299 |
| V.15 | $C_3H_7$ | $CH_2CH=CHCH_3$ | 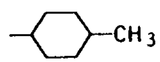 | H | H | H | EP-A 88 299 |
| V.16 | $C_2H_5$ | $CH_2CH=CHCH_3$ | 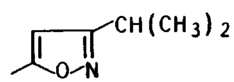 | H | H | H | EP-A 238 021 |
| V.17 | $C_3H_7$ | $CH_2CH=CHCH_3$ | 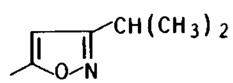 | H | H | H | EP-A 238 021 |
| V.18 | $C_2H_5$ | $CH_2CH=CHCl$ | 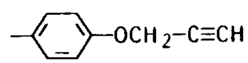 | H | H | H | EP-A 137 174 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.19 | $C_3H_7$ | $C_2H_5$ | 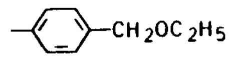 | H | H | H | EP-A 2 137 200 |
| V.20 | $C_3H_7$ | $C_2H_5$ | 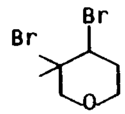 | H | H | H | EP-A 230 235 |
| V.21 | $C_3H_7$ | $CH_2CH=CHCl$ | 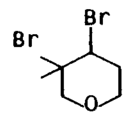 | H | H | H | EP-A 230 235 |
| V.22 | $C_3H_7$ | $CH_2CH=CHCl$ | 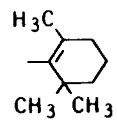 | H | H | H | EP-A 46 860 |
| V.23 | $C_3H_7$ | $C_2H_5$ | 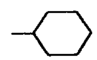 | H | H | H | JP-A 540 191 945 |
| V.24 | $C_3H_7$ | $C_2H_5$ | 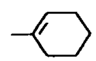 | H | H | H | EP-A 46 860 |
| V.25 | $CH_3$ | $CH_2CH=CHCl$ | 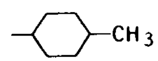 | H | H | H | EP-A 88 299 |
| V.26 | $C_3H_7$ | $C_2H_5$ | 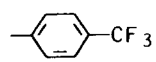 | H | H | K | EP-A 137 174 |
| V.27 | $C_2H_5$ | $CH_2CH=CHCl$ | 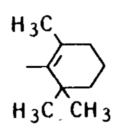 | H | H | H | EP-A 46 860 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.28 | $C_3H_7$ | $CH_2CH{=}CHCH_3$ | 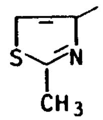 | H | H | H | EP-A 125 094 |
| V.29 | $C_3H_7$ | $CH_2CH{=}CHCl$ | 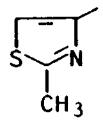 | H | H | H | EP-A 125 094 |
| V.30 | $C_3H_7$ | $C_2H_5$ | 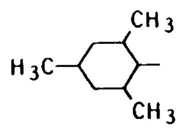 | H | H | H | EP-A 88 299 |
| V.31 | $C_3H_7$ | $H_2CH{=}CH_2$ | 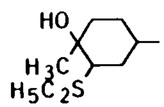 | H | H | H | EP-A 228 598 |
| V.32 | $C_2H_5$ | $C_2H_5$ | 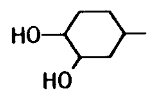 | H | H | H | EP-A 228 598 |
| V.33 | $C_3H_7$ | $C_2H_5$ | 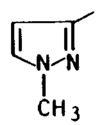 | H | H | H | EP-A 66 195 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.34 | $C_3H_7$ | $CH_2CH{=}CHCl$ | 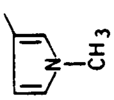 | H | H | H | EP-A 66195 |
| V.35 | $C_3H_7$ | $CH_2CH{=}CH_2$ | 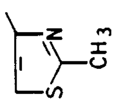 | H | H | H | EP-A 125 094 |
| V.36 | $C_3H_7$ | $C_3H_7$ | $CH(SCH_2CH_3)_2$ | H | H | H | EP-A 230 260 |
| V.37 | $C_3H_7$ | $C_2H_5$ | 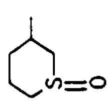 | H | H | H | EP-A 115 808 |
| V.38 | $C_3H_7$ | $C_2H_5$ | 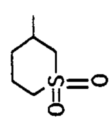 | H | H | H | EP-A 115 808 |
| V.39 | $C_3H_7$ | $C_2H_5$ | $CH_3$ | $CH_3$ | $C(CH_3){=}NOCH_3$ | H | EP-A 172 551 |
| V.40 | $C_3H_7$ | $CH_2CH{=}CH_2$ | 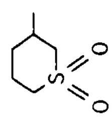 | OH | H | H | Proceedings Brit. Crop-Protection Conference - weeds 1985 vol.1 S. 93-98 |

Herbizide Wirkstoffe und antidotisch wirkende Verbindungen können gleichzeitig oder nacheinander in beliebiger Reihenfolge vor, bei oder nach der Aussaat der Kulturpflanzen, vor oder während des Auflaufens der Kulturpflanzen, nach dem Auflaufen auf die Blätter und Sprosse der Kulturpflanzen und der unerwünschten Gräser ausgebracht werden. Bevorzugt wird das antidotisch wirkende Mittel gleichzeitig mit dem herbiziden Wirkstoff ausgebracht. Auch eine getrennte Ausbringung, wobei das Antidot zuerst und anschließend der herbizide Wirkstoff auf das Feld gebracht werden, ist möglich. Herbizider Wirkstoff und Antidot können hierbei als Spritzmittel in suspendierbarer, emulgierbarer oder löslicher Form gemeinsam oder getrennt formuliert vorliegen.

Denkbar ist auch eine Behandlung der Kulturpflanzensamen mit dem Antidot vor der Aussaat. Der herbizide Wirkstoff wird dann allein in der üblichen Weise appliziert.

Für herbizide (Heteroaryloxy)phenoxysäurederivate werden unterschiedliche Mengen einer antidotisch wirkenden Verbindung benötigt, wenn das Herbizid in verschiedenen Kulturen eingesetzt wird. Die Mengenverhältnisse sind in breiten Bereichen variabel. Sie sind ebenfalls abhängig von der Struktur der (Heteroaryloxy)phenoxysäurederivate und der jeweiligen Zielkultur. Geeignete Anteilverhältnisse herbizider Wirkstoffe: antidotisch wirkender Verbindung liegen zwischen 1 : 10 bis 1 : 0,01; vorzugsweise zwischen 1 : 4 bis 1 : 0,1 Gew.-Teile.

Für das gleiche Cyclohexenonderivat werden unterschiedliche Mengen einer antidotisch wirkenden Verbindung benötigt, wenn das Cyclohexenonderivat in verschiedenen Kulturen eingesetzt wird. Die Mengenverhältnisse, in denen ein Cyclohexenonderivat und ein 1,8-Naphthyridinderivat I eingesetzt werden, sind in breiten Bereichen variabel. Sie sind abhängig von der Struktur des Cyclohexenonderivats, des Naphthyridinderivates I und der jeweiligen Kultur. Geeignete Anteilverhältnisse herbizider Wirkstoff: antidotisch wirkender Verbindung liegen zwischen 1 : 10 bis 1 : 0,01; vorzugsweise 1 : 4 bis 1 : 0,25 Gew.-Teile.

Die neuen herbiziden Mittel können neben dem Naphthyridinderivat I als Antidot und dem Herbizid aus der Gruppe der (Heteroaryloxy)phenoxyessigsäuren IV bzw. der Cyclohexenone V weitere herbizide oder wachstumsregulierende Wirkstoffe anderer chemischer Struktur enthalten, wobei der antagonistische Effekt erhalten bleibt.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentige wäßrige, ölige oder sonstige Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Ölsdispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon und Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin-Sulfitablaugen und Methylcellulose in Betracht.

Pulver, Streu- und Stäubmittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gew.-% an herbizidem Wirkstoff und Antidot, vorzugsweise zwischen 0,5 und 90 Gew.-%. Die Aufwandmengen an herbizidem Wirkstoff betragen 0,2 bis 5 kg/ha.

Die vorliegende Erfindung wird durch die nachstehend beschriebenen Beispiele weiter erläutert.

Beispiel 1

2-Amin-5-chlormethyl-7-hydroxy-1,8-naphthyridin

Zu einer Suspension von 21,8 g (0,2 mol) 2,6-Diaminopyridin in 100 ml 85%iger Phosphorsäure tropft man 32,9 g (0,2 mol) 4-Chloracetessigsäureethylester. Anschließend rührt man die Reaktionsmischung noch 3 Stunden lang bei 90 °C, kühlt ab, gießt auf Eiswasser und neutralisiert mit konzentrierter wäßriger Ammoniak-Lösung. Der ausgefallene Niederschlag wird abgesaugt, nochmals mit Wasser verrührt und schließlich mit Acetonitril ausgekocht. Es verbleiben 22,8 g (54 %) der Titelverbindung: Fp. >280 °C.

Beispiel 2

2-Amin-5-methoxymethyl-7-hydroxy-1,8-naphthyridin

Eine Mischung aus 21,8 g (0,2 mol) 2,6-Diaminopyridin und 29,2 g (0,2 mol) 4-Methoxyacetessigsäuremethylester in 400 ml Eisessig wird 3 Stunden lang bei Rückfluß gerührt. Nach dem Abkühlen wird der ausgefallene Niederschlag abgesaugt und mit 150 ml kaltem Ethanol verrührt. Es verbleiben 8 g (20 %) der Titelverbindung: Fp. >290 °C.

Beispiel 3

Acetylierung von 2-Amino-5,7-dihydroxy-1,8-naphthyridin

Eine Mischung aus 17,7 g (0,1 mol) des nach Lit.[3]) dargestellten 2-Amino-5,7-dihydroxy-1,8-naphthyridins und 250 ml Acetanhydrid wird 30 Minuten lang bei Rückfluß gerührt. Nach dem Erkalten wird der ausgefallene Niederschlag abgesaugt, mit Aceton gewaschen und getrocknet. Man erhält 20,9 g (80 %) einer Mischung aus 2-Acetylamino-5-hydroxy-7-acetoxy-1,8-naphthyridin und 2-Acetylamino-5-acetoxy-7-hydroxy-1,8-naphthyridin: Fp. >300 °C.

Diese Mischung kann direkt in die nachfolgend beschriebene Chlorierung mit $POCl_3$ eingesetzt werden.

Beispiel 4

2-Amino-5,7-dichlor-1,8-naphthyridin

Eine Suspension aus 63 g (0,241 mol) des nach Beispiel 3 dargestellten Gemisches und 600 ml $POCl_3$ wird langsam zum Rückfluß erhitzt und dann noch 2 Stunden lang unter Rückfluß gerührt. Anschließend wird der auf etwa die Hälfte eingeengte Ansatz unter Eiskühlung vorsichtig in 2,5 l Eiswasser eingetropft. Die nunmehr resultierende Mischung wird eine Stunde bei 95-100 °C gerührt, anschließend abgekühlt, vom Rückstand abfiltriert und das erhaltene Filtrat auf pH = 6 gebracht. Der ausgefallene Niederschlag wird abgesaugt, mit warmem Wasser gründlich ausgewaschen und getrocknet. Man erhält 47,9 g (92 %) der Titelverbindung mit dem Schmelzpunkt 213-216 °C.

Beispiel 5

2-Amino-5-methyl-7-methylthio-1,8-naphthyridin

Zu einer Lösung von 112 g (1,6 mol) Natriumthiomethanolat in 700 ml Wasser gibt man 77,5 g (0,4 mol) des nach Lit.[5]) dargestellten 2-Amino-5-methyl-7-chlor-1,8-naphthyridins und erhitzt die resultierende Suspension 4 Stunden unter Rückfluß. Nach dem Abkühlen wird der Rückstand abgesaugt, mit Wasser gewaschen und getrocknet. Man erhält 68 g (83 %) der Titelverbindung mit dem Schmelzpunkt 204-209 °C.

Beispiel 6

2-Hydroxysulfonyl-5,7-dimethyl-1,8-naphthyridin

Zu einer mit verdünnter wäßriger Salzsäure auf pH = 7-8 eingestellten Lösung von 8,2 g (65 mmol) Natriumsulfit in 40 ml Wasser gibt man 9,6 g (50 mmol) des nach Literatur 8), 9) hergestellten 2-Chlor-5,7-dimethyl-1,8-naphthyridins und erhitzt die so erhaltene Suspension 12 h unter Rückfluß. Nach dem Abkühlen werden feste Rückstände aus der Lösung entfernt und die Lösung selbst wird mit wäßriger Salzsäure angesäuert (pH etwa 1). Man beläßt die angesäuerte Lösung 12 h bei 0 °C bis 5 °C, wobei das Produkt auskristallisiert. Man erhält 7,7 g (65 %) der Titelverbindung: Fp. >280 °C.

Die in der anschließenden Tabelle 1 aufgeführten Verbindungen wurden aus entsprechenden Ausgangsstoffen unter entsprechender Abwandlung der Versuchsangaben der Beispiele 1-6 hergestellt.

Tabelle 1

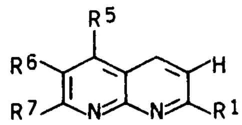

| Beispiel Nr. | $R^1$ | $R^5$ | $R^6$ | $R^7$ | Fp (°C) | Lit.[a)] |
|---|---|---|---|---|---|---|
| 1.1 | $HNCOCH_3$ | OH | H | H | 264-265 | 10) |
| 1.2 | $NH_2$ | Cl | H | Cl | 213-216 | |
| 1.3 | $NH_2$ | OH | H | H | >300 | 10) |
| 1.4 | $NH_2$ | $CH_3$ | H | H | >300 | 1) |
| 1.5 | $HNCOCH_3$ | Phenyl | H | $CH_3$ | 207-213 | 5) |
| 1.6 | $HNCOCH_3$ | $CH_3$ | H | $OC_6H_5$ | 203-205 | |
| 1.7 | $NH_2$ | OH | $CO_2H$ | H | >300 | 10) |
| 1.8 | $NH_2$ | H | H | Cl | >300 | |
| 1.9 | $HNCOCH_3$ | OH | $CO_2C_2H_5$ | H | >300 | 10) |
| 1.10 | $NH_2$ | $CH_2Cl$ | H | Cl | >280 | |
| 1.11 | $NH_2$ | $CH_2OCH_3$ | H | OH | >290 | |
| 1.12 | $HNCOCH_3$ | H | H | OH | >280 | 11) |
| 1.13 | $NH_2$ | H | H | $CH_3$ | 238 (Zers.) | 12) |
| 1.14 | $NH_2$ | H | H | OH | >280 | 2), 11) |
| 1.15 | $NH_2$ | $C_6H_5$ | H | $CH_3$ | 246 (Zers.) | 4) |
| 1.16 | $NH_2$ | $CH_3$ | H | $SCH_3$ | 204-209 | |
| 1.17 | $NH_2$ | $CH_3$ | H | $OC_6H_5$ | 206-210 | 5) |
| 1.18 | $NH_2$ | $CH_3$ | H | $C_6H_5$ | >280 | 1), 4) |
| 1.19 | $HNCOCH_3$ | $CH_2Cl$ | H | OH | >280 | |
| 1.20 | $NH_2$ | $CH_2Cl$ | H | OH | >280 | |
| 1.21 | $NH_2$ | Br | H | Br | 208-212 | |
| 1.22 | $NH_2$ | $CH_3$ | H | Br | >300 | |
| 1.23 | $NH_2$ | $CH_3$ | H | $N(CH_3)_2$ | >300 | |
| 1.24 | $NH_2$ | H | $CO_2C_2H_5$ | OH | >300 | 11) |
| 1.25 | SH | $CH_3$ | H | $CH_3$ | 272-274 | |

Tabelle 1

| Beispiel Nr. | $R^1$ | $R^5$ | $R^6$ | $R^7$ | Fp (°C) | Lit.[a] |
|---|---|---|---|---|---|---|
| 1.26 | $NH_2$ | $CF_3$ | H | $CF_3$ | 204-208 | 13) |
| 1.27 | $NH_2$ | OH | H | OH | 325 (Zers.) | 3) |
| 1.28 | $SCH_2C_6H_5$ | $CH_3$ | H | $CH_3$ | 98-104 | |
| 1.29 | $NH_2$ | $CH_3$ | H | $OCH_3$ | 229-232 | |
| 1.30 | $NH_2$ | $CH_3$ | H | Cl | 256-261 | 5) |
| 1.31 | $HNCOCH_3$ | $CH_3$ | H | Cl | 238-240 | 5) |
| 1.32 | $OCH_3$ | $CH_3$ | H | $CH_3$ | 37-40 | 9) |
| 1.33 | Cl | $CH_3$ | H | $CH_3$ | 138-141 | 8), 9) |
| 1.34 | $HNCOCH_3$ | $CH_3$ | H | OH | 395 | 5) |
| 1.35 | OH | $CH_3$ | H | $CH_3$ | 253-257 | 8), 9) |
| 1.36 | $NH_2$ | $CH_3$ | H | OH | >300 | 6) |
| 1.37 | $NH_2$ | $CH_3$ | H | $CH_3$ | 218-220 | 7) |
| 1.38 | $NH_2$ | $CF_3$ | H | $CH_3$ | 185-187 | 13) |
| 1.39 | $NH_2$ | $CH_3$ | H | $CF_3$ | 244-246 | 13) |
| 1.40 | $NH_2$ | $OCH_3$ | H | $OCH_3$ | 230-232 | 13) |
| 1.41 | $SO_3H$ | $CH_3$ | H | $CH_3$ | >280 | |

[a] Literaturquellen


1) R.A. Henry und P.R. Hammond, J. Het. Chem. 14, 1109 (1977)

2) S. Carboni, A. Da Settimo und G. Pirisino, Ann. di Chim. 54, 883 (1964)

3) G. R. Lappin, Q.R. Petersen u. C.E. Wheeler, J. Org. Chem. 15, 377 (1950)

4) A. Mangini u. M. Colonna, Gazz. chim. ital. 73, 330 (1943)

5) V. Petrow, E. L. Rewald u. B. Sturgeon, J. Chem.Soc. London, 1407 (1947)

6) O. Seide, Chem. Ber. LIX, 159 (1926).

7) J. Bernstein et al., J. Am. Chem. Soc. 69, 1157 (1947)

8) A. Mangini u. M. Colonna, Gazz. chim. ital. 73, 323 (1943)

9) E. Ochiai u. K. Miyaki, Chem. Ber. 74, 1115 (1941)

10) S. Carboni, A. Da Settimo, P.L. Ferrarini u. I. Tonetti, Gazz. chim. ital. 101, 129 (1971)

11) S. Carboni, A. Da Settimo u. P.L. Ferrarini, Gazz. chim. ital. 95, 1492 (1965)

12) E.V. Brown, J. Org. Chem. 30, 1607 (1965)

13) E. Eichler, C.S. Rooney u. H.W.R. Williams, J. Het. Chem. 13, 41 (1976)

Beispiel 7

2-Amino-6-brom-5,7-dimethyl-1,8-naphthyridin

173 g (1 mol) 2-Amino-5,7-dimethyl-1,8-naphthyridin werden in 500 ml Eisessig suspendiert. Bei 60 °C werden unter Rühren 160 g (1 mol) Brom zugetropft, wobei der Feststoff in Lösung geht. Nach beendeter Zugabe wird noch über Nacht bei Raumtemperatur (20 °C) gerührt und dann in 1 l Wasser gegossen. Überschüssiger Brom wird durch Zugabe von wenig $NaHSO_3$ reduziert. Mit konzentrierter $NH_3$-Lösung wird auf pH = 4 gebracht. Man rührt noch ca. 30 Minuten nach, saugt den ausgefallenen Niederschlag ab, trocknet im Vakuum und erhält so 132 g eines hellbraunen Feststoffs.

Beispiel 8

2-Amino-5,6-dimethyl-7-phenylthio-1,8-naphthyridin

5,0 g (24 mmol) 2-Amino-7-chlor-5,6-dimethyl-1,8-naphthyridin und 8,0 g (72 mmol) Thiophenol werden in 100 ml trockenem Methanol vorgelegt. Nach Zugabe von 13 g einer 30 %igen methanolischen Natriummethylatlösung kocht man drei Stunden unter Rückfluß und rührt über Nacht bei Raumtemperatur. Dann gibt man 200 ml Wasser zu, rührt 1 h nach und saugt den ausgefallenen Feststoff ab, der mit wenig Methanol gewaschen und im Vakuum getrocknet wird. Man erhält so 4,7 g eines blaßgelben Feststoffs.

Beispiel 9

2-Acetamino-7-hydroxy-5,6-dimethyl-1,8-naphthyridin

142 g (0,75 mol) 2-Amino-7-hydroxy-5,6-dimethyl-1,8-naphthyridin und 640 ml Acetanhydrid werden 30 Minuten unter Rückfluß gekocht. Nach Abkühlen wird das Produkt abgesaugt und durch Ausrühren mit $CH_2Cl_2$ und methanol gereinigt. Man erhält so 64,1 g (37 %) eines grauen Feststoffs. $^1$H-NMR (200 MHz, ($F_3$-($O_2$D): $\delta$ = 2.42 (s, 3H, $CH_3$), 2.58 (s, 3H, $CH_3$), 2.69 (s, 3H, $CH_3$), 7.46 (d, 1H, Ar-H), 8.84 (d, 1H, Ar-H).

Beispiel 10

2-Amino-7-Chlor-5,6-dimethyl-1,8-naphthyridin

34,6 g (0,15 mol) 2-Acetamino-7-hydroxy-5,6-dimethyl-1,8-naphthyridin und 380 ml Phosphoroxichlorid werden 30 Minuten unter Rückfluß gekocht, wobei das Edukt in Lösung geht. Überschüssiges $POCl_3$ wird abdestilliert, der Rückstand auf Eis gegeben und dann mit konz. Salzsäure versetzt. Nach 30 Minuten Kochen unter Rückfluß wird mit 50%iger Natronlauge alkalisch gestellt. Der ausgefallene Niederschlag wird abgesaugt und aus 4 l Methanol umkristallisiert. Man erhält so 28,7 g (92 %) eines hellbraunen Feststoffs,

der ohne weitere Reinigung weiter umgesetzt wird.

Die in der nachfolgenden Tabelle 2 aufgeführten Verbindungen wurden aus entsprechenden Ausgangsstoffen unter entsprechender Abwandlung der Beispiele 7, 8, 9 und 10 hergestellt.

Tabelle 2

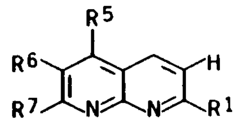

| Beisp. Nr. | $R^1$ | $R^5$ | $R^6$ | $R^7$ | Fp (°C) | $^1$H-NMR (200 MHz, DMSO-$D_6$) δ [ppm] | Lit.[a)] |
|---|---|---|---|---|---|---|---|
| 2.1 | $NH_2$ | Cl | $CH_3$ | Cl | 150 (Z) | 2.55 (s, 3H, $CH_3$), 7.07 (d,1H, Ar-H), 7.85 (s (br.), 2H, $NH_2$), 8.23 (d,1H, Ar-H). | |
| 2.2 | $NH_2$ | Cl | i-$C_3H_7$ | Cl | >145 (Z) | 1.42 (d, 6H, $CH_3$), 3.90 (n,1H, $CH(CH_3)_2$), 7.10 (d,1H, Ar-H), 8.10 (s (br.), 2H, $NH_2$), 8.30 (d,1H, Ar-H). | |
| 2.3 | $NH_2$ | Cl | n-$C_4H_9$ | Cl | 220-225 | 0.94 (t, 3H, $CH_3$), 1.46 (m, 4H, $(CH_2)_2$), 2.94 (t, 2H, Ar-$CH_2$, 7.34 (d,1H, Ar-H), 8.46 (d,1H,Ar-H) 8.85 (s. (br.), 2H, $NH_2$). | |
| 2.4 | $NH_2$ | OH | i-$C_3H_7$ | OH | >250 | 1.28 (d, 6H, $CH_3$), 3.38 (h, 1H, $(CH_3)_2CH$), 6.33 (d, 1H, Ar-H), 7.30 (s (br.), 2H, $NH_2$), 7.94 (d, 1H, Ar-H), 9.95 (s (br.), 1H, OH, 11.79 (s, 1H, OH). | |
| 2.5 | $NH_2$ | OH | $CH_3$ | OH | >200 | | |
| 2.6 | $NH_2$ | $CH_3$ | $CH_3$ | $CH_3$ | >230 | 2.27 (s, 3H, $CH_3$), 2.45 (s, 3H, $CH_3$), 2.52 (s, 3H, $CH_3$), 6.49 (s, 2H, $NH_2$), 6.23 (d, 1H, Ar-H), 8.09 (d, 1H, Ar-H). | 14),15) |
| 2.7 | $NH_2$ | $CH_3$ | Cl | $CH_3$ | | 2.55 (s, 3H, $CH_3$), 2.60 (s, 3H, $CH_3$), 5.90 (s (br.), 2H, $NH_2$), 6.82 (d, 1H, Ar-H), 8.13 (d, 1H, Ar-H). | |
| 2.8 | $NH_2$ | $CH_3$ | Br | $CH_3$ | | 2.61 (s, 3H, $CH_3$), 2.63 (s, 3H, $CH_3$), 6.85 (d, 1H, Ar-H), 7.2 (s (br.), 2H, $NH_2$), 8.10 (d, 1H, Ar-H). | |

Tabelle 2 - Fortsetzung -

| Beisp. Nr. | $R^1$ | $R^5$ | $R^6$ | $R^7$ | Fp (°C) | 1H-NMR (200 MHz, DMSO-$D_6$) δ [ppm] | Lit.[a)] |
|---|---|---|---|---|---|---|---|
| 2.9 | $NH_2$ | $CH_3$ | $CH_3$ | OH | >190 (Z) | 2.5 (s, 6H, $CH_3$), 4.5 (s (br.), 2H, $NH_2$), 6.0 (d, 1H, Ar-H), 7.38 (d, 1H, Ar-H). | 16 |
| 2.10 | $NH_2$ | $CH_3$ | i-$C_3H_7$ | OH | | 1.50 (d, 6H, $CH_3$), 2.77 (s, 3H, Ar-$CH_3$), 7.17 (d, 1H, Ar-H, 8.42 (d, 1H, Ar-H). (in Trifluoressigsäure) | |
| 2.11 | $NH_2$ | $CH_3$ | $CH_3$ | Cl | braunes Öl | | |
| 2.12 | $NH_2$ | $CH_3$ | i-$C_3H_7$ | Cl | braunes Öl | | |
| 2.13 | $NH_2$ | $CH_3$ | $CH_3$ | $OCH_3$ | | 2.18 (s, 3H, Ar-$CH_3$), 2.40 (s, 3H, Ar-$CH_3$), 3.95 (s, 3H, $OCH_3$), 6.60 (s, 2H, $NH_2$), 6.63 (d, 1H, Ar-H), 7.99 (d, 1H, Ar-H). | |
| 2.14 | $NH_2$ | $CH_3$ | $CH_3$ | $SC_6H_5$ | | 2.32 (s, 3H, $CH_3$), 2.44 (s, 3H, $CH_3$), 6.65 (s, 2H, $NH_2$), 6.70 (d, 1H, Ar-H), 7.45-7.6 (m, 5H, s-Ph), 8.10 (d, 1H, Ar-H). | |
| 2.15 | $NHCOCH_3$ | $CH_3$ | $CH_3$ | OH | | 2.42 (s, 3H, $CH_3$), 2.58 (s, 3H, $CH_3$), 2.69 (s, 3H, $CH_3$), 7.46 (d, 1H, Ar-H), 8.84 (d, 1H, Ar-H) | |

Tabelle 2 - Fortsetzung -

| Beisp. Nr. | $R^1$ | $R^5$ | $R^6$ | $R^7$ | Fp (°C) | $^1$H-NMR (200 MHz, DMSO-$D_6$) δ [ppm] | Lit.[a] |
|---|---|---|---|---|---|---|---|
| 2.16 | NHCO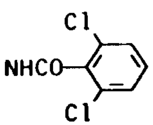 | $CH_3$ | H | $CH_3$ | 287-291 | | |
| 2.17 | $NHCOC_6H_5$ | OH | $CO_2CH_2CH_3$ | H | 235-240 | | |

(Z): schmilzt unter Zersetzung

[a]Literatur: 14) US-A 4 133 885
15) W.A. Bolhofer et al. J. Med. Chem. 22 (3), 301 (1979)
16) CA-A 1 088 659

Beispiele zur biologischen Wirkung

Der Einfluß verschiedener Vertreter der erfindungsgemäßen herbiziden Mittel bzw. Mittelkombinationen, bestehend aus Herbizid und antidotisch wirkender Verbindung, auf das Wachstum von erwünschten und

unerwünschten Pflanzen im Vergleich zum herbiziden Wirkstoff allein wird durch die folgenden biologischen Beispiele aus Gewächshausversuchen belegt:

Als Kulturgefäße dienten Plastiktöpfe mit rund 300 $cm^3$ Inhalt und lehmiger Sand mit etwa 3,0 % Humus als Substrat. Die Samen der Testpflanzen werden nach Arten getrennt, flach eingesät und befeuchtet. Danach wurden die Gefäße mit durchsichtigen Plastikhauben abgedeckt, bis die Samen gleichmäßig gekeimt und die Pflanzen angewachsen waren.

Für die Nachauflaufbehandlung wurden die Testpflanzen je nach Wuchsform erst bis zu einer Wuchshöhe von 3 bis 20 cm gezüchtet und erst dann behandelt. Die herbiziden Mittel wurden hierbei in Wasser als Verteilungsmittel suspendiert oder emulgiert und mittels fein verteilender Düsen gespritzt.

Als Beispielsherbizid der Cyclohexenonderivate der Formel V diente

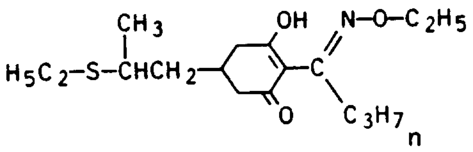

V.2

Der herbizide Wirkstoff v.2 wurde als kommerziell formuliertes Produkt (184 g/l EC auch allein jeweils unter Zugabe von derjenigen Menge an Lösungsmittelsystem XXII - Leerformulierung -) in die Spritzbrühe gegeben und gemeinsam mit der antidotischen Verbindung ausgebracht.

Sämtliche antidotisch wirkende Verbindungen wurden für die Nachauflaufbehandlung in einem Gemisch, bestehend aus 80 % Cyclohexenon und 20 % Emulphor EL (Versuchsformulierung XXII) mit 10 % (Gew.-%) Wirkstoff aufbereitet.

| Liste der Testpflanzen | | |
|---|---|---|
| Lateinischer Name | Deutscher Name | Englischer Name |
| Hordeum vulgaris | Gerste | barley |
| Lolium multiflorum | Ital. Raygrass | annual ryegras |
| Oryza sativa | Reis | rice |
| Setaria italica | Ital. Raygras | italian ryegras |
| Sorghum | Hirse | millet |
| Triticum aestivum | Weizen | wheat |
| Zea mays | Mais | corn |

Die Versuchsgefäße wurden im Gewächshaus aufgestellt, wobei für wärmeliebende Arten 18 bis 30 °C und für solche gemäßigtere Klimate (10 bis 25 °C) bevorzugt wurden.

Die Versuchsperiode erstreckte sich über 3 bis 5 Wochen. Während dieser Zeit wurden die Pflanzen gepflegt, und ihre Reaktion auf die einzelnen Behandlungen wurde erfaßt.

Bewertet wurde die Schädigung durch die chemischen Mittel anhand einer Skala von 0 bis 100 % im Vergleich zu den unbehandelten Kontrollpflanzen. Dabei bedeutet 0 keine Schädigung und 100 eine völlige Zerstörung der Pflanzen.

Die anschließenden Tabellen dokumentieren die antidotische Wirkung der erfindungsgemäßen Beispielsverbindungen Nr. 1.10, 1.16, 1.8, 1.17 und 2.13.

Dabei verbessern diese Beispielsverbindungen deutlich die Verträglichkeit des Herbizids V.2 für Weizen.

Tabelle 1

| Verbesserung der Verträglichkeit des Herbizids V.2 für Weizen durch Zumischen einer antidotischen Beispielsverbindung und Nachauflaufanwendung im Gewächshaus | | | |
|---|---|---|---|
| Aufwandmenge kg/ha a.S. | | Testpflanzen und Schädigung % Kulturpflanze unerwünschte | |
| Herbizid V.2 | Antidot 1.10 | Triticum aestivum* | Pflanze Lolium multiflorum |
| 0,03 | - | 65 | 100 |
| 0,06 | - | 95 | 100 |
| 0,03 | 0,125 | 0 | 100 |
| 0,06 | 0,25 | 30 | 100 |

*Sorte: "Okapi"

Tabelle 2

| Verringerung der Phytotoxizität des Herbizids V.2 gegenüber Weizen durch Kombination mit einer antidotischen Beispielsverbindung und Nachauflaufanwendung im Gewächshaus | | | |
|---|---|---|---|
| Aufwandmenge kg/ha a.S. | | Testpflanzen und Schädigung % Kulturpflanze unerwünschte | |
| Herbizid V.2 | Antidot 1.16 | Triticum aestivum* | Pflanze Lolium multiflorum |
| 0,03 | - | 65 | 100 |
| 0,03 | 0,125 | 0 | 100 |

*Sorte: "Okapi"

Tabelle 3

| Verringerung der Phytotoxizität des Herbizids V.2 gegenüber Weizen durch Kombination mit einer antidotischen Beispielsverbindung und Nachauflaufanwendung im Gewächshaus | | | |
|---|---|---|---|
| Aufwandmenge kg/ha a.S. | | Testpflanzen und Schädigung % Kulturpflanze unerwünschte | |
| Herbizid V.2 | Antidot [Nr.] | Triticum aestivum* | Pflanze Lolium multiflorum |
| 0,03 | - | 65 | 100 |
| 0,03 | 0,125 [1.8] | 30 | 100 |
| 0,03 | 0,125 [1.17] | 20 | 100 |

*Sorte: "Okapi"

Tabelle 4

| Verringerung der Phytotoxizität des Herbizids V.2 gegenüber Reis durch Kombination mit der Beispielverbindung 2.13 bei Nachauflaufanwendung im Gewächshaus | | | |
|---|---|---|---|
| Aufwandmenge [kg/ha a.S.] | | Testpflanzen und Schädigung % Kulturpflanze unerwünschte Pflanze | |
| Herbizid | Antidot | Oryza sativa* | Setaria italica |
| 0.03 | | 50 | 95 |
| 0.03 | 0.06 | 10 | 80 |

* Sorte: "Bahia"

## Patentansprüche

**Patentansprüche für folgende Vertagsstaaten : BE, CH, DE, DK, FR, GB, IT, LI, NL**

**1.** Substituierte 1,8-Naphthyridine der allgemeinen Formel I

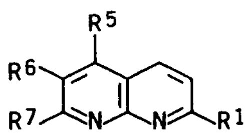

I,

in der die Substituenten die folgende Bedeutung haben:

$R^1$ Mercapto, Benzylthio, Sulfonyl, Amino oder $NR^2R^3$, wobei

$R^2$ $C_1$-$C_{12}$-Alkyl, $C_3$-$C_6$-Alkenyl, $C_3$-$C_8$-Cycloalkyl, Phenyl, Phenyl-($C_1$-$C_3$)-alkyl, ein- bis dreifach durch Halogen, $C_1$-$C_4$-Alkyl, $C_1$-$C_4$-Alkoxi, Amino, Mono- oder Dialkylamino mit 1 bis 6 C-Atomen je Alkylrest substituiertes Phenyl oder Phenyl-($C_1$-$C_3$)-alkyl oder eine Gruppe

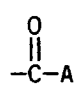

,

worin A für $C_1$-$C_8$-Alkyl, Phenyl, ein- bis dreifach durch Halogen, $C_1$-$C_4$-Alkyl, $C_1$-$C_4$-Alkoxy, Amino, Mono- oder Dialkylamino mit 1 bis 6 C-Atomen je Alkylrest substituiertes Phenyl, Amino, Alkyl- oder Dialkylamino mit jeweils 1 bis 6 C-Atomen je Alkylrest, Phenylamino, Morpholino oder Piperidyl steht, und

$R^3$ Wasserstoff, $C_1$-$C_{12}$-Alkyl, $C_3$-$C_8$-Cycloalkyl bedeuten oder

$R^2$ und $R^3$ miteinander zu einem gesättigten oder ungesättigten 5- oder 6-gliedrigen Heterocyclus, der zusätzlich zu dem Stickstoffatom, an sie gebunden sind ein weiteres Heteroatom wie Sauerstoff oder Stickstoff enthalten kann, verbunden sind;

Hydroxyl, Mercapto, Halogen oder

$XR^4$, worin X Sauerstoff oder Schwefel bedeutet und $R^4$ für $C_1$-$C_{12}$-Alkyl, $C_1$-$C_4$-Alkoxicarbonyl-($C_1$-$C_4$)-alkyl, $C_5$-$C_8$-Cycloalkyl, Phenyl oder Phenyl-$C_1$-$C_3$-alkyl, wobei die aromatischen Ringe ein bis drei der folgenden Reste tragen können: Halogen, $C_1$-$C_4$-Alkyl, $C_1$-$C_4$-Alkoxi, Amino, Mono- oder Dialkylamino mit 1 bis 6 C-Atomen je Alkylrest,

bedeutet,

$R^5$, Wasserstoff, $C_1$-$C_{12}$-Alkyl, $C_3$-$C_8$-Cycloalkyl,

$R^7$ Phenyl oder Phenyl-($C_1$-$C_3$)-alkyl, wobei der Phenylkern jeweils seinerseits ein bis drei der folgenden Gruppen tragen kann: Halogen, $C_1$-$C_4$-Alkyl, $C_1$-$C_4$-Alkoxy, $C_1$-$C_4$-Halogenalkyl, Amino oder Mono- oder Dialkylamino mit 1 bis 4 C-Atomen je Alkylrest; Hydroxy, Halogen, Mercapto, $C_1$-$C_4$-Alkoxy, $C_1$-$C_4$-Alkylthio, Amino, Mono- oder Dialkylamino mit 1 bis 6 C-Atomen je Alkylrest, Phenoxy oder Phenylthio, wobei der Phenylkern jeweils seinerseits ein bis drei der folgenden Gruppen tragen kann: Halogen, $C_1$-$C_4$-Alkyl, $C_1$-$C_4$-Alkoxy, $C_1$-$C_4$-Halogenalkyl, Amino oder Mono- oder Dialkylamino mit 1 bis 4 C-Atomen je Alkylrest; Halogen-($C_1$-$C_3$)-alkyl, $C_1$-$C_4$-Alkoxy-($C_1$-$C_3$)-alkyl, $C_1$-$C_4$-Alkylthio-($C_1$-$C_3$)-alkyl, Amino-($C_1$-$C_3$)-alkyl, $C_1$-$C_4$-Acyloxy, welches ein bis drei $C_1$-$C_4$-Alkylreste und/oder einen $C_1$-$C_4$-Acylrest tragen kann; Hydrazino, Cyano und Thiocyanato,

$R^6$ Wasserstoff, $C_1$-$C_{12}$-Alkyl, $C_3$-$C_8$-Cycloalkyl, Phenyl oder Phenyl-($C_1$-$C_3$)-alkyl, wobei der Phenylkern jeweils seinerseits ein bis drei der folgenden Gruppen tragen kann: Halogen, $C_1$-$C_4$-Alkyl, $C_1$-$C_4$-Alkoxy, $C_1$-$C_4$-Halogenalkyl, Amino oder Mono- oder Dialkylamino mit 1 bis 4 C-Atomen je Alkylrest; Halogen, Halogen-($C_1$-$C_3$)-alkyl, $C_1$-$C_4$-Alkoxy-($C_1$-$C_3$)-alkyl, $C_1$-$C_4$-Alkylthio-($C_1$-$C_3$)-alkyl, Amino-($C_1$-$C_3$)-alkyl, Carboxyl, $C_1$-$C_4$-Alkoxycarbonyl,

wobei $R^1$ nicht Amino bedeutet, wenn $R^5$ und $R^6$ für Wasserstoff und $R^7$ für Wasserstoff oder Phenyl steht,

sowie deren pflanzenverträgliche Salze.

**2.** Verfahren zur Herstellung der Verbindungen I gemäß Anspruch 1, dadurch gekennzeichnet, daß man 2,6-Diaminopyridin der Formel II

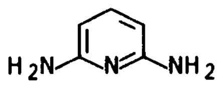

II

mit einer $\beta$-Dicarbonylverbindung der allgemeinen Formel III

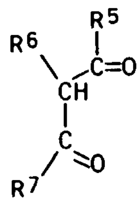

III

umsetzt und gegebenenfalls anschließend eine Derivatisierung oder Substitution der Reste $R^1$ und/oder $R^5$ und/oder $R^6$ und/oder $R^7$ in an sich bekannter Weise vornimmt.

**3.** Herbizide Mittel, enthaltend mindestens ein substituiertes 1,8-Naphthyridin der allgemeinen Formel I gemäß Anspruch 1 oder dessen pflanzenverträgliche Salze und mindestens einen herbiziden Wirkstoff aus der Gruppe

a) der 2-(4-Heteroaryloxy)- oder 2-(4-Aryloxy)-phenoxyessigsäurederivate der Formel IV,

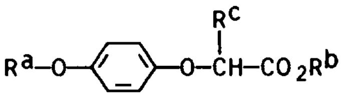

IV

in der die Substituenten folgende Bedeutung haben:

$R^a$ ein Phenylring, ein Pyridylring, ein Benzoxazylrest, ein Benzthiazylrest oder ein Benzpyrazinylrest, wobei diese aromatischen Ringsysteme bis zu zwei der folgenden Reste tragen können: Halogen, Nitro, $C_1$-$C_4$-Alkyl, $C_1$-$C_4$-Halogenalkyl und/oder $C_1$-$C_4$-Halogenalkoxy,

$R^b$ Wasserstoff, eine $C_1$-$C_4$-Alkylgruppe oder das Äquivalent eines pflanzenverträglichen Kations und

$R^c$ Wasserstoff oder eine Methylgruppe

und

b)

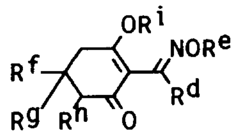

V

in welcher die Substituenten folgende Bedeutung haben:

$R^d$ eine $C_1$-$C_4$-Alkylgruppe;

$R^e$ eine $C_1$-$C_4$-Alkylgruppe, eine $C_3$-$C_4$-Alkenylgruppe, eine $C_3$-$C_4$-Alkinylgruppe, eine $C_3$-$C_4$-Halogenalkylengruppe oder eine Thenylgruppe, welche durch ein Halogenatom substituiert sein kann;

$R^f$ eine $C_1$-$C_4$-Alkylgruppe, welche ein- oder zweifach durch $C_1$-$C_4$-Alkylthio oder $C_1$-$C_4$-Alkoxy substituiert sein kann;
ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, und dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;
ein 10-gliedriger gesättigter oder einfach ungesättigter Heterocyclus, welcher zwei Sauerstoffatome oder Schwefelatome enthält und durch bis zu drei $C_1$-$C_4$-Alkylgruppen und/oder Methoxygruppen substituiert sein kann;
ein Phenylrest, ein Pyridylrest ein Thiazolylrest, ein Pyrazolylrest, ein Pyrrolylrest oder ein Isoxazolylrest, wobei diese Reste bis zu drei der folgenden Gruppen tragen können: $C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und/oder Benzoylamino

$R^g$ Wasserstoff, Hydroxy oder, wenn $R^f$ die Bedeutung $C_1$-$C_6$-Alkylgruppe hat, eine $C_1$-$C_6$-Alkylgruppe;

$R^h$ Wasserstoff, eine Cyanogruppe, ein Halogenatom eine $C_1$-$C_4$-Alkoxycarbonylgruppe oder eine $C_1$-$C_4$-Alkylketoximgruppe und

$R^i$ Wasserstoff oder das Äquivalent eines umweltverträglichen Kations.

**4.** Herbizides Mittel nach Anspruch 3, enthaltend ein substituiertes 1,8-Naphthyridin I gemäß Anspruch 3 und ein Herbizid a) oder ein Herbizid b), wobei das Anteilsverhältnis 10:1 bis 0,01:1 Gew.-Teile beträgt.

**5.** Verfahren zur selektiven Bekämpfung von unerwünschtem Pflanzenwuchs, dadurch gekennzeichnet, daß man ein substituiertes 1,8-Naphthyridin der Formel I gemäß Anspruch 3 und ein 2-(4-Heteroaryloxy)- oder 2-(4-Aryloxy)-phenoxyessigsäurederivat der Formel IV oder ein Cyclohexenonderivat der Formel V gemäß Anspruch 3 vor, bei oder nach der Aussaat der Kulturpflanzen, vor oder während des Auflaufens der Kulturpflanzen gleichzeitig oder nacheinander in beliebiger Reihenfolge ausbringt.

**6.** Verfahren zur Verhinderung von Schädigungen von Kulturpflanzen durch herbizide 2-(4-Heteroaryloxy)- oder 2-(4-Aryloxy)-phenoxyessigsäurederivate der Formel IV oder herbizide Cyclohexenonderivate der Formel V gemäß Anspruch 3, dadurch gekennzeichnet, daß man das Saatgut der Kulturpflanzen mit einer antagonistisch wirksamen Menge eines substituierten 1,8-Naphthyridins der Formel I gemäß Anspruch 3 behandelt.

**7.** Verfahren zur selektiven Bekämpfung von unerwünschtem Pflanzenwuchs, dadurch gekennzeichnet, daß man die Blätter der Kulturpflanzen und der unerwünschten Pflanzen im Nachauflaufverfahren mit einem substituierten 1,8-Naphthyridin der Formel I gemäß Anspruch 3 und mit einem 2-(4-Heteroaryloxy)- oder 2-(4-Aryloxy)-phenoxyessigsäurederivat der Formel IV oder einem Cyclohexenonderivat der Formel V gemäß Anspruch 3 gleichzeitig oder nacheinander behandelt.

**8.** Verfahren gemäß den Ansprüchen 5 bis 7, dadurch gekennzeichnet, daß die Kulturpflanzen Gerste, Weizen, Mais, Kultursorghum und Reis sind.

**Patentansprüche für folgenden Vertragsstaat : ES**

**1.** Verfahren zur Herstellung von substituierten 1,8-Naphthyridinen der allgemeinen Formel I

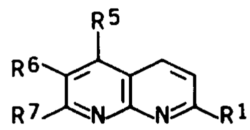

I,

in der die Substituenten die folgende Bedeutung haben:

$R^1$ Mercapto, Benzylthio, Sulfonyl, Amino oder $NR^2R^3$, wobei

$R^2$ $C_1$-$C_{12}$-Alkyl, $C_3$-$C_6$-Alkenyl, $C_3$-$C_8$-Cycloalkyl, Phenyl, Phenyl-($C_1$-$C_3$)-alkyl, ein- bis dreifach durch Halogen, $C_1$-$C_4$-Alkyl, $C_1$-$C_4$-Alkoxi, Amino, Mono- oder Dialkylamino mit 1 bis 6 C-Atomen je Alkylrest substituiertes Phenyl oder Phenyl-($C_1$-$C_3$)-alkyl oder eine Gruppe

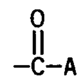

,

worin A für $C_1$-$C_8$-Alkyl, Phenyl, ein- bis dreifach durch Halogen, $C_1$-$C_4$-Alkyl, $C_1$-$C_4$-Alkoxy, Amino, Mono- oder Dialkylamino mit 1 bis 6 C-Atomen je Alkylrest substituiertes Phenyl, Amino, Alkyl- oder Dialkylamino mit jeweils 1 bis 6 C-Atomen je Alkylrest, Phenylamino, Morpholino oder Piperidyl steht, und

$R^3$ Wasserstoff, $C_1$-$C_{12}$-Alkyl, $C_3$-$C_8$-Cycloalkyl bedeuten oder

$R^2$ und $R^3$ miteinander zu einem gesättigten oder ungesättigten 5- oder 6-gliedrigen Heterocyclus, der zusätzlich zu dem Stickstoffatom, an sie gebunden sind ein weiteres Heteroatom wie Sauerstoff oder Stickstoff enthalten kann, verbunden sind;

Hydroxyl, Mercapto, Halogen oder

$XR^4$, worin X Sauerstoff oder Schwefel bedeutet und $R^4$ für $C_1$-$C_{12}$-Alkyl, $C_1$-$C_4$-Alkoxicarbonyl-($C_1$-$C_4$)-alkyl, $C_5$-$C_8$-Cycloalkyl, Phenyl oder Phenyl-$C_1$-$C_3$-alkyl, wobei die aromatischen Ringe ein bis drei der folgenden Reste tragen können: Halogen, $C_1$-$C_4$-Alkyl, $C_1$-$C_4$-Alkoxi, Amino, Mono- oder Dialkylamino mit 1 bis 6 C-Atomen je Alkylrest,

bedeutet,

$R^5$, Wasserstoff, $C_1$-$C_{12}$-Alkyl, $C_3$-$C_8$-Cycloalkyl,

$R^7$ Phenyl oder Phenyl-($C_1$-$C_3$)-alkyl, wobei der Phenylkern jeweils seinerseits ein bis drei der folgenden Gruppen tragen kann: Halogen, $C_1$-$C_4$-Alkyl, $C_1$-$C_4$-Alkoxy, $C_1$-$C_4$-Halogenalkyl, Amino oder Mono- oder Dialkylamino mit 1 bis 4 C-Atomen je Alkylrest; Hydroxy, Halogen, Mercapto, $C_1$-$C_4$-Alkoxy, $C_1$-$C_4$-Alkylthio, Amino, Mono- oder Dialkylamino mit 1 bis 6 C-Atomen je Alkylrest, Phenoxy oder Phenylthio, wobei der Phenylkern jeweils seinerseits ein bis drei der folgenden Gruppen tragen kann: Halogen, $C_1$-$C_4$-Alkyl, $C_1$-$C_4$-Alkoxy, $C_1$-$C_4$-Halogenalkyl, Amino oder Mono- oder Dialkylamino mit 1 bis 4 C-Atomen je Alkylrest; Halogen-($C_1$-$C_3$)-alkyl, $C_1$-$C_4$-Alkoxy-($C_1$-$C_3$)-alkyl, $C_1$-$C_4$-Alkylthio-($C_1$-$C_3$)-alkyl, Amino-($C_1$-$C_3$)-alkyl, $C_1$-$C_4$-Acyloxy, welches ein bis drei $C_1$-$C_4$-Alkylreste und/oder einen $C_1$-$C_4$-Acylrest tragen kann; Hydrazino, Cyano und Thiocyanato,

$R^6$ Wasserstoff, $C_1$-$C_{12}$-Alkyl, $C_3$-$C_8$-Cycloalkyl, Phenyl oder Phenyl-($C_1$-$C_3$)-alkyl, wobei der Phenylkern jeweils seinerseits ein bis drei der folgenden Gruppen tragen kann: Halogen, $C_1$-$C_4$-Alkyl, $C_1$-$C_4$-Alkoxy, $C_1$-$C_4$-Halogenalkyl, Amino oder Mono- oder Dialkylamino mit 1 bis 4 C-Atomen je Alkylrest; Halogen, Halogen-($C_1$-$C_3$)-alkyl, $C_1$-$C_4$-Alkoxy-($C_1$-$C_3$)-alkyl, $C_1$-$C_4$-Alkylthio-($C_1$-$C_3$)-alkyl, Amino-($C_1$-$C_3$)-alkyl, Carboxyl, $C_1$-$C_4$-Alkoxycarbonyl,

wobei $R^1$ nicht Amino bedeutet, wenn $R^5$ und $R^6$ für Wasserstoff und $R^7$ für Wasserstoff oder Phenyl steht,

sowie deren pflanzenverträgliche Salze,

dadurch gekennzeichnet, daß man 2,6-Diaminopyridin der Formel II

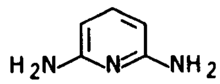

II

mit einer β-Dicarbonylverbindung der allgemeinen Formel III

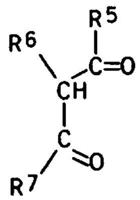

III

umsetzt und gegebenenfalls anschließend eine Derivatisierung oder Substitution der Reste $R^1$ und/oder $R^5$ und/oder $R^6$ und/oder $R^7$ in an sich bekannter Weise vornimmt.

**2.** Herbizide Mittel, enthaltend mindestens ein substituiertes 1,8-Naphthyridin der allgemeinen Formel I gemäß Anspruch 1 oder dessen pflanzenverträgliche Salze und mindestens einen herbiziden Wirkstoff aus der Gruppe

a) der 2-(4-Heteroaryloxy)- oder 2-(4-Aryloxy)-phenoxyessigsäurederivate der Formel IV,

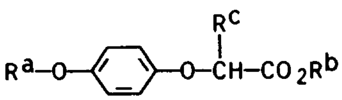

IV

in der die Substituenten folgende Bedeutung haben:

$R^a$ ein Phenylring, ein Pyridylring, ein Benzoxazylrest, ein Benzthiazylrest oder ein Benzpyrazinylrest, wobei diese aromatischen Ringsysteme bis zu zwei der folgenden Reste tragen können: Halogen, Nitro, $C_1$-$C_4$-Alkyl, $C_1$-$C_4$-Halogenalkyl und/oder $C_1$-$C_4$-Halogenalkoxy,

$R^b$ Wasserstoff, eine $C_1$- $C_4$-Alkylgruppe oder das Äquivalent eines pflanzenverträglichen Kations und

$R^c$ Wasserstoff oder eine Methylgruppe

und

b)

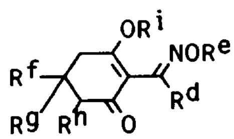

V

in welcher die Substituenten folgende Bedeutung haben:

$R^d$ eine $C_1$-$C_4$-Alkylgruppe;

$R^e$ eine $C_1$-$C_4$-Alkylgruppe, eine $C_3$-$C_4$-Alkenylgruppe, eine $C_3$-$C_4$-Alkinylgruppe, eine $C_3$-$C_4$-Halogenalkylengruppe oder eine Thenylgruppe, welche durch ein Halogenatom substituiert sein kann;

$R^f$ eine $C_1$-$C_4$-Alkylgruppe, welche ein- oder zweifach durch $C_1$-$C_4$-Alkylthio oder $C_1$-$C_4$-Alkoxy substituiert sein kann;
ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, und dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;
ein 10-gliedriger gesättigter oder einfach ungesättigter Heterocyclus, welcher zwei Sauerstoffatome oder Schwefelatome enthält und durch bis zu drei $C_1$-$C_4$-Alkylgruppen und/oder

Methoxygruppen substituiert sein kann;
ein Phenylrest, ein Pyridylrest ein Thiazolylrest, ein Pyrazolylrest, ein Pyrrolylrest oder ein Isoxazolylrest, wobei diese Reste bis zu drei der folgenden Gruppen tragen können: $C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und/oder Benzoylamino

$R^g$ Wasserstoff, Hydroxy oder, wenn $R^f$ die Bedeutung $C_1$-$C_6$-Alkylgruppe hat, eine $C_1$-$C_6$-Alkylgruppe;

$R^h$ Wasserstoff, eine Cyanogruppe, ein Halogenatom eine $C_1$-$C_4$-Alkoxycarbonylgruppe oder eine $C_1$-$C_4$-Alkylketoximgruppe und

$R^i$ Wasserstoff oder das Äquivalent eines umweltverträglichen Kations.

**3.** Herbizides Mittel nach Anspruch 2, enthaltend ein substituiertes 1,8-Naphthyridin I gemäß Anspruch 2 und ein Herbizid a) oder ein Herbizid b), wobei das Anteilsverhältnis 10:1 bis 0,01:1 Gew.-Teile beträgt.

**4.** Verfahren zur selektiven Bekämpfung von unerwünschtem Pflanzenwuchs, dadurch gekennzeichnet, daß man ein substituiertes 1,8-Naphthyridin der Formel I gemäß Anspruch 2 und ein 2-(4-Heteroaryloxy)- oder 2-(4-Aryloxy)-phenoxyessigsäurederivat der Formel IV oder ein Cyclohexenonderivat der Formel V gemäß Anspruch 2 vor, bei oder nach der Aussaat der Kulturpflanzen, vor oder während des Auflaufens der Kulturpflanzen gleichzeitig oder nacheinander in beliebiger Reihenfolge ausbringt.

**5.** Verfahren zur Verhinderung von Schädigungen von Kulturpflanzen durch herbizide 2-(4-Heteroaryloxy)- oder 2-(4-Aryloxy)-phenoxyessigsäurederivate der Formel IV oder herbizide Cyclohexenonderivate der Formel V gemäß Anspruch 2, dadurch gekennzeichnet, daß man das Saatgut der Kulturpflanzen mit einer antagonistisch wirksamen Menge eines substituierten 1,8-Naphthyridins der Formel I gemäß Anspruch 2 behandelt.

**6.** Verfahren zur selektiven Bekämpfung von unerwünschtem Pflanzenwuchs, dadurch gekennzeichnet, daß man die Blätter der Kulturpflanzen und der unerwünschten Pflanzen im Nachauflaufverfahren mit einem substituierten 1,8-Naphthyridin der Formel I gemäß Anspruch 2 und mit einem 2-(4-Heteroaryloxy)- oder 2-(4-Aryloxy)-phenoxyessigsäurederivat der Formel IV oder einem Cyclohexenonderivat der Formel V gemäß Anspruch 2 gleichzeitig oder nacheinander behandelt.

**7.** Verfahren gemäß den Ansprüchen 4 bis 6, dadurch gekennzeichnet, daß die Kulturpflanzen Gerste, Weizen, Mais, Kultursorghum und Reis sind.

**Claims**
**Claims for the following Contracting States : BE, CH, DE, DK, FR, GB, IT, LI, NL**

**1.** A substituted 1,8-naphthyridine of the general formula I

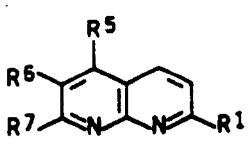

I

where

$R^1$ is mercapto, benzylthio, sulfonyl, amino or $NR^2R^3$, $R^2$ is $C_1$-$C_{12}$-alkyl, $C_3$-$C_6$-alkenyl, $C_3$-$C_8$-cycloalkyl, phenyl, phenyl-$C_1$-$C_3$-alkyl or phenyl or phenyl-$C_1$-$C_3$-alkyl which is monosubstituted to trisubstituted by halogen, $C_1$-$C_4$-alkyl, $C_1$-$C_4$-alkoxy, amino or mono- or dialkylamino where each alkyl radical is of 1 to 6 carbon atoms, or a group

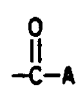

,

where A is $C_1$-$C_8$-alkyl or is phenyl which is unsubstituted or monosubstituted to trisubstituted by halogen, $C_1$-$C_4$-alkyl, $C_1$-$C_4$-alkoxy, amino or mono- or dialkylamino where each alkyl radical is of 1 to 6 carbon atoms, or is amino, alkylamino or dialkylamino where each alkyl radical is of 1 to 6 carbon atoms, phenylamino, morpholino or piperidyl, and $R^3$ is hydrogen, $C_1$-$C_{12}$-alkyl or $C_3$-$C_8$-cycloalkyl, or $R^2$ and $R^3$ are bonded to one another to form a saturated or unsaturated 5-membered or 6-membered heterocyclic structure which, in addition to the nitrogen atom to which they are bonded, may contain a further heteroatom, such as oxygen or nitrogen; hydroxyl, mercapto, halogen or $XR^4$ where X is oxygen or sulfur and $R^4$ is $C_1$-$C_{12}$-alkyl, $C_1$-$C_4$-alkoxycarbonyl-$C_1$-$C_4$-alkyl, $C_5$-$C_8$-cycloalkyl, phenyl or phenyl-$C_1$-$C_3$-alkyl, where the aromatic rings may carry from one to three of the following radicals: halogen, $C_1$-$C_4$-alkyl, $C_1$-$C_4$-alkoxy, amino or mono- or dialkylamino where each alkyl radical is of 1 to 6 carbon atoms,

$R^5$ and $R^7$ are each hydrogen, $C_1$-$C_{12}$-alkyl, $C_3$-$C_8$-cycloalkyl, phenyl or phenyl-$C_1$-$C_3$-alkyl, where the phenyl nucleus in each case may in turn carry from one to three of the following groups: halogen, $C_1$-$C_4$-alkyl, $C_1$-$C_4$-alkoxy, $C_1$-$C_4$-haloalkyl, amino or mono- or dialkylamino where each alkyl radical is of 1 to 4 carbon atoms; hydroxyl, halogen, mercapto, $C_1$-$C_4$-alkoxy, $C_1$-$C_4$-alkylthio, amino, mono- or dialkylamino where each alkyl radical is of 1 to 6 carbon atoms, phenoxy or phenylthio, where the phenyl nucleus in each case may in turn carry from one to three of the following groups: halogen, $C_1$-$C_4$-alkyl, $C_1$-$C_4$-alkoxy, $C_1$-$C_4$-haloalkyl, amino or mono- or dialkylamino where each alkyl radical is of 1 to 4 carbon atoms; halo-$C_1$-$C_3$-alkyl, $C_1$-$C_4$-alkoxy-$C_1$-$C_3$-alkyl, $C_1$-$C_4$-alkylthio-$C_1$-$C_3$-alkyl, amino-$C_1$-$C_3$-alkyl, $C_1$-$C_4$-acyloxy which may carry from one to three $C_1$-$C_4$-alkyl radicals and/or a $C_1$-$C_4$-acyl radical; hydrazino, cyano or thiocyanato, and

$R^6$ is hydrogen, $C_1$-$C_{12}$-alkyl, $C_3$-$C_8$-cycloalkyl, phenyl or phenyl-$C_1$-$C_3$-alkyl, where the phenyl nucleus in each case may in turn carry from one to three of the following groups: halogen, $C_1$-$C_4$-alkyl, $C_1$-$C_4$-alkoxy, $C_1$-$C_4$-haloalkyl, amino or mono- or dialkylamino where each alkyl radical is of 1 to 4 carbon atoms; halogen, halo-$C_1$-$C_3$-alkyl, $C_1$-$C_4$-alkoxy-$C_1$-$C_3$-alkyl, $C_1$-$C_4$-alkylthio-$C_1$-$C_3$-alkyl, amino-$C_1$-$C_3$-alkyl, carboxyl or $C_1$-$C_4$-alkoxycarbonyl,

$R^1$ not being amino when $R^5$ and $R^6$ are each hydrogen and $R^7$ is hydrogen or phenyl, or a plant-tolerated salt thereof.

2. A process for the preparation of a compound I as claimed in claim 1, which comprises reacting a 2,6-diaminopyridine of the formula II

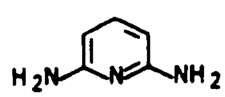

II

with a $\beta$-dicarbonyl compound of the general formula III

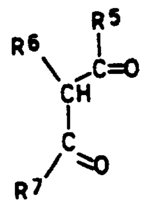

III

and then, if appropriate, carrying out a derivatization or substitution of $R^1$ and/or $R^5$ and/or $R^6$ and/or $R^7$ in a conventional manner.

3. A herbicidal composition containing at least one substituted 1,8-naphthyridine of the general formula I as claimed in claim 1, or a plant-tolerated salt thereof, and at least one herbicidal active ingredient selected from the group consisting of

a) the 2-(4-hetaryloxy)- or 2-(4-aryloxy)-phenoxyacetic acid derivatives of the formula IV

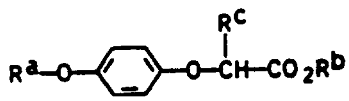

IV

where

$R^a$ is a phenyl ring, a pyridyl ring, a benzoxazyl radical, a benzothiazyl radical or a benzopyrazinyl radical, and these aromatic ring systems may carry up to two of the following radicals: halogen, nitro, $C_1$-$C_4$-alkyl, $C_1$-$C_4$-haloalkyl and/or $C_1$-$C_4$-haloalkoxy,

$R^b$ is hydrogen, $C_1$-$C_4$-alkyl or one equivalent of a plant-tolerated cation and

$R^c$ is hydrogen or methyl,

and

b)

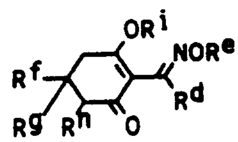

V

where

$R^d$ is $C_1$-$C_4$-alkyl;

$R^e$ is $C_1$-$C_4$-alkyl, $C_3$- or $C_4$-alkenyl, $C_3$- or $C_4$-alkynyl, $C_3$- or $C_4$-haloalkylene or thenyl which may be substituted by a halogen atom;

$R^f$ is $C_1$-$C_4$-alkyl which may be monosubstituted or disubstituted by $C_1$-$C_4$-alkylthio or $C_1$-$C_4$-alkoxy;

a 5-membered or 6-membered saturated or mono-unsaturated ring system which, in addition to carbon members, may contain an oxygen or sulfur atom or a sulfoxyl or sulfonyl group, and this ring may carry up to three of the following radicals: hydroxyl, halogen, $C_1$-$C_4$-alkyl, $C_1$-$C_4$-haloalkyl, $C_1$-$C_4$-alkoxy and/or $C_1$-$C_4$-alkylthio;

a 10-membered saturated or monounsaturated heterocyclic structure which contains two oxygen atoms or sulfur atoms and may be substituted by up to three $C_1$-$C_4$-alkyl groups and/or methoxy groups;

a phenyl, pyridyl, thiazolyl, pyrazolyl, pyrrolyl or isoxazolyl radical which may carry up to three of the following groups: $C_1$-$C_4$-alkyl, $C_1$-$C_4$-haloalkyl, $C_1$-$C_4$-alkoxy, $C_1$-$C_4$-alkylthio, $C_3$-$C_6$-alkenyloxy, $C_3$-$C_6$-alkynyloxy, $C_1$-$C_4$-alkoxy-$C_1$-$C_3$-alkyl, $C_1$-$C_4$-dialkoxy-$C_1$-$C_3$-alkyl, formyl, halogen and/or benzoylamino,

$R^g$ is hydrogen or hydroxyl or, when $R^f$ is $C_1$-$C_6$-alkyl, is $C_1$-$C_6$-alkyl;

$R^h$ is hydrogen, cyano, halogen, $C_1$-$C_4$-alkoxycarbonyl or a $C_1$-$C_4$-alkylketoxime group and

$R^i$ is hydrogen or one equivalent of an environmentally compatible cation.

**4.** A herbicidal composition as claimed in claim 3, containing a substituted 1,8-naphthyridine I as claimed in claim 3 and a herbicide a) or a herbicide b), the weight ratio being from 10:1 to 0.01:1.

**5.** A method for selectively controlling unwanted plant growth, wherein a substituted 1,8-naphthyridine of the formula I as claimed in claim 3 and a 2-(4-hetaryloxy)- or 2-(4-aryloxy)-phenoxyacetic acid derivative of the formula IV or a cyclohexenone derivative of the formula V as claimed in claim 3 are applied either simultaneously or one after the other in any order before, during or after sowing of the crop plants, or before or during emergence of the crop plants.

**6.** A method for preventing damage to crop plants by herbicidal 2-(4-hetaryloxy)- or 2-(4-aryloxy)-phenoxyacetic acid derivatives of the formula IV or herbicidal cyclohexenone derivatives of the formula V as claimed in claim 3, wherein the seed of the crop plants is treated with an antagonistic amount of a substituted 1,8-naphthyridine of the formula I as claimed in claim 3.

7. A method for selectively controlling undesirable plant growth, wherein the leaves of the crop plants and of the undesirable plants are treated postemergence by simultaneous or successive application of a substituted 1,8-naphthyridine of the formula I as claimed in claim 3 and with a 2-(4-hetaryloxy)- or 2-(4-aryloxy)-phenoxyacetic acid derivative of the formula IV or with a cyclohexenone derivative of the formula V as claimed in claim 3.

8. A method as claimed in any of claims 5 to 7 wherein the crop plants are barley, wheat, Indian corn, sorghum and rice.

**Claims for the following Contracting State : ES**

1. A process for the preparation of a substituted 1,8-naphthyridine of the general formula I

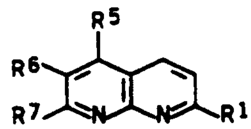

I

where

$R^1$ is mercapto, benzylthio, sulfonyl, amino or $NR^2R^3$, $R^2$ is $C_1$-$C_{12}$-alkyl, $C_3$-$C_6$-alkenyl, $C_3$-$C_8$-cycloalkyl, phenyl, phenyl-$C_1$-$C_3$-alkyl or phenyl or phenyl-$C_1$-$C_3$-alkyl which is monosubstituted to trisubstituted by halogen, $C_1$-$C_4$-alkyl, $C_1$-$C_4$-alkoxy, amino or mono- or dialkylamino where each alkyl radical is of 1 to 6 carbon atoms, or a group

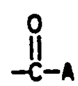

,

where A is $C_1$-$C_8$-alkyl or is phenyl which is unsubstituted or monosubstituted to trisubstituted by halogen, $C_1$-$C_4$-alkyl, $C_1$-$C_4$-alkoxy, amino or mono- or dialkylamino where each alkyl radical is of 1 to 6 carbon atoms, or is amino, alkylamino or dialkylamino where each alkyl radical is of 1 to 6 carbon atoms, phenylamino, morpholino or piperidyl, and $R^3$ is hydrogen, $C_1$-$C_{12}$-alkyl or $C_3$-$C_8$-cycloalkyl, or $R^2$ and $R^3$ are bonded to one another to form a saturated or unsaturated 5-membered or 6-membered heterocyclic structure which, in addition to the nitrogen atom to which they are bonded, may contain a further heteroatom, such as oxygen or nitrogen; hydroxyl, mercapto, halogen or $XR^4$
where X is oxygen or sulfur and $R^4$ is $C_1$-$C_{12}$-alkyl, $C_1$-$C_4$-alkoxycarbonyl-$C_1$-$C_4$-alkyl, $C_5$-$C_8$-cycloalkyl, phenyl or phenyl-$C_1$-$C_3$-alkyl, where the aromatic rings may carry from one to three of the following radicals: halogen, $C_1$-$C_4$-alkyl, $C_1$-$C_4$-alkoxy, amino or mono- or dialkylamino where each alkyl radical is of 1 to 6 carbon atoms,

$R^5$ and $R^7$ are each hydrogen, $C_1$-$C_{12}$-alkyl, $C_3$-$C_8$-cycloalkyl, phenyl or phenyl-$C_1$-$C_3$-alkyl, where the phenyl nucleus in each case may in turn carry from one to three of the following groups: halogen, $C_1$-$C_4$-alkyl, $C_1$-$C_4$-alkoxy, $C_1$-$C_4$-haloalkyl, amino or mono- or dialkylamino where each alkyl radical is of 1 to 4 carbon atoms; hydroxyl, halogen, mercapto, $C_1$-$C_4$-alkoxy, $C_1$-$C_4$-alkylthio, amino, mono- or dialkylamino where each alkyl radical is of 1 to 6 carbon atoms, phenoxy or phenylthio, where the phenyl nucleus in each case may in turn carry from one to three of the following groups: halogen, $C_1$-$C_4$-alkyl, $C_1$-$C_4$-alkoxy, $C_1$-$C_4$-haloalkyl, amino or mono- or dialkylamino where each alkyl radical is of 1 to 4 carbon atoms; halo-$C_1$-$C_3$-alkyl, $C_1$-$C_4$-alkoxy-$C_1$-$C_3$-alkyl, $C_1$-$C_4$-alkylthio-$C_1$-$C_3$-alkyl, amino-$C_1$-$C_3$-alkyl, $C_1$-$C_4$-acyloxy which may carry from one to three $C_1$-$C_4$-alkyl radicals and/or a $C_1$-$C_4$-acyl radical; hydrazino, cyano or thiocyanato, and

$R^6$ is hydrogen, $C_1$-$C_{12}$-alkyl, $C_3$-$C_8$-cycloalkyl, phenyl or phenyl-$C_1$-$C_3$-alkyl, where the phenyl nucleus in each case may in turn carry from one to three of the following groups: halogen, $C_1$-$C_4$-alkyl, $C_1$-$C_4$-alkoxy, $C_1$-$C_4$-haloalkyl, amino or mono- or

dialkylamino where each alkyl radical is of 1 to 4 carbon atoms; halogen, halo-$C_1$-$C_3$-alkyl, $C_1$-$C_4$-alkoxy-$C_1$-$C_3$-alkyl, $C_1$-$C_4$-alkylthio-$C_1$-$C_3$-alkyl, amino-$C_1$-$C_3$-alkyl, carboxyl or $C_1$-$C_4$-alkoxycarbonyl,

$R^1$ not being amino when $R^5$ and $R^6$ are each hydrogen and $R^7$ is hydrogen or phenyl, or a plant-tolerated salt thereof,

which comprises reacting a 2,6-diaminopyridine of the formula II

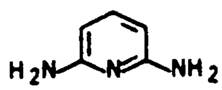

II

with a $\beta$-dicarbonyl compound of the general formula III

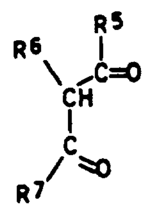

III

and then, if appropriate, carrying out a derivatization or substitution of $R^1$ and/or $R^5$ and/or $R^6$ and/or $R^7$ in a conventional manner.

**2.** A herbicidal composition containing at least one substituted 1,8-naphthyridine of the general formula I as claimed in claim 1, or a plant-tolerated salt thereof, and at least one herbicidal active ingredient selected from the group consisting of

a) the 2-(4-hetaryloxy)- or 2-(4-aryloxy)-phenoxyacetic acid derivatives of the formula IV

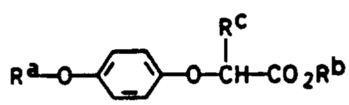

IV

where

$R^a$ is a phenyl ring, a pyridyl ring, a benzoxazyl radical, a benzothiazyl radical or a benzopyrazinyl radical, and these aromatic ring systems may carry up to two of the following radicals: halogen, nitro, $C_1$-$C_4$-alkyl, $C_1$-$C_4$-haloalkyl and/or $C_1$-$C_4$-haloalkoxy,

$R^b$ is hydrogen, $C_1$-$C_4$-alkyl or one equivalent of a plant-tolerated cation and

$R^c$ is hydrogen or methyl,

and

b)

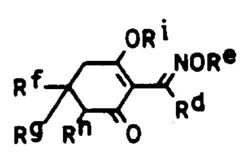

V

where

$R^d$ is $C_1$-$C_4$-alkyl;

$R^e$ is $C_1$-$C_4$-alkyl, $C_3$- or $C_4$-alkenyl, $C_3$- or $C_4$-alkynyl, $C_3$- or $C_4$-haloalkylene or thenyl which may be substituted by a halogen atom;

$R^f$ is $C_1$-$C_4$-alkyl which may be monosubstituted or disubstituted by $C_1$-$C_4$-alkylthio or $C_1$-$C_4$-

alkoxy;
a 5-membered or 6-membered saturated or mono-unsaturated ring system which, in addition to carbon members, may contain an oxygen or sulfur atom or a sulfoxyl or sulfonyl group, and this ring may carry up to three of the following radicals: hydroxyl, halogen, $C_1$-$C_4$-alkyl, $C_1$-$C_4$-haloalkyl, $C_1$-$C_4$-alkoxy and/or $C_1$-$C_4$-alkylthio;
a 10-membered saturated or monounsaturated heterocyclic structure which contains two oxygen atoms or sulfur atoms and may be substituted by up to three $C_1$-$C_4$-alkyl groups and/or methoxy groups;
a phenyl, pyridyl, thiazolyl, pyrazolyl, pyrrolyl or isoxazolyl radical which may carry up to three of the following groups: $C_1$-$C_4$-alkyl, $C_1$-$C_4$-haloalkyl, $C_1$-$C_4$-alkoxy, $C_1$-$C_4$-alkylthio, $C_3$-$C_6$-alkenyloxy, $C_3$-$C_6$-alkynyloxy, $C_1$-$C_4$-alkoxy-$C_1$-$C_3$-alkyl, $C_1$-$C_4$-dialkoxy-$C_1$-$C_3$-alkyl, formyl, halogen and/or benzoylamino,
$R^g$ is hydrogen or hydroxyl or, when $R^f$ is $C_1$-$C_6$-alkyl, is $C_1$-$C_6$-alkyl;
$R^h$ is hydrogen, cyano, halogen, $C_1$-$C_4$-alkoxycarbonyl or a $C_1$-$C_4$-alkylketoxime group and
$R^i$ is hydrogen or one equivalent of an environmentally compatible cation.

**3.** A herbicidal composition as claimed in claim 2, containing a substituted 1,8-naphthyridine I as claimed in claim 2 and a herbicide a) or a herbicide b), the weight ratio being from 10:1 to 0.01:1.

**4.** A method for selectively controlling unwanted plant growth, wherein a substituted 1,8-naphthyridine of the formula I as claimed in claim 2 and a 2-(4-hetaryloxy)- or 2-(4-aryloxy)-phenoxyacetic acid derivative of the formula IV or a cyclohexenone derivative of the formula V as claimed in claim 2 are applied either simultaneously or one after the other in any order before, during or after sowing of the crop plants, or before or during emergence of the crop plants.

**5.** A method for preventing damage to crop plants by herbicidal 2-(4-hetaryloxy)- or 2-(4-aryloxy)-phenoxyacetic acid derivatives of the formula IV or herbicidal cyclohexenone derivatives of the formula V as claimed in claim 2, wherein the seed of the crop plants is treated with an antagonistic amount of a substituted 1,8-naphthyridine of the formula I as claimed in claim 2.

**6.** A method for selectively controlling undesirable plant growth, wherein the leaves of the crop plants and of the undesirable plants are treated postemergence by simultaneous or successive application of a substituted 1,8-naphthyridine of the formula I as claimed in claim 2 and with a 2-(4-hetaryloxy)- or 2-(4-aryloxy)-phenoxyacetic acid derivative of the formula IV or with a cyclohexenone derivative of the formula V as claimed in claim 2.

**7.** A method as claimed in any of claims 4 to 6 wherein the crop plants are barley, wheat, Indian corn, sorghum and rice.

## Revendications

**Revendications pour les Etats contractants suivants : BE, CH, DE, DK, FR, GB, IT, LI, NL**

**1.** 1,8-naphtyridines substituées répondant à la formule généralé

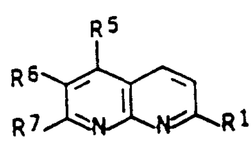

I,

dans laquelle les symboles ont les significations suivantes :
$R^1$ représente un groupe mercapto, benzylthio, sulfonyle, amino ou $NR^2R^3$ dans lequel $R^2$ représente un groupe alkyle en C1-C12, alcényle en C3-C6, cycloalkyle en C3-C8, phényle, phényl-alkyle en C1-C3, un groupe phényle ou phényl-alkyle en C1-C3 portant un à trois substituants choisis parmi les halogènes, les groupes alkyle en C1-C4, alcoxy en C1-C4, amino, mono- ou di-alkylamino contenant 1 à 6 atomes de carbone par groupe alkyle ; ou bien un groupe

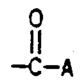,

dans lequel A représente un groupe alkyle en C1-C8, phényle, un groupe phényle portant 1 à 3 substituants choisis parmi les halogènes, les groupes alkyle en C1-C4, alcoxy en C1-C4, amino, mono- ou di-alkylamino contenant 1 à 6 atomes de carbone par groupe alkyle, un groupe amino, alkyl- ou dialkyl-amino contenant 1 à 6 atomes de carbone par groupe alkyle, phénylamino, morpholino ou pipéridyle, et

$R^3$ représente l'hydrogène, un groupe alkyle en C1-C12, cycloalkyle en C3-C8, ou bien

$R^2$ et $R^3$ représentent ensemble un hétérocycle saturé ou insaturé à 5 ou 6 chaînons qui, en plus de l'atome d'azote auquel ils sont réliés, peut contenir un autre hétéroatome tel que l'oxygène ou l'azote ;

un groupe hydroxy, marcapto, un halogène ou bien

un groupe $XR^4$ dans lequel X représente l'oxygène ou le soufre et $R^4$ un groupe alkyle en C1-C12, (alcoxy en C1-C4)-carbonyl-alkyle en C1-C4, cycloalkyle en C5-C8, phényle ou phényl-alkyle en C1-C3 dont les noyaux aromatiques peuvent porter 1 à 3 des substituants suivants :

des halogènes, des groupes alkyle en C1-C4, alcoxy en C1-C4, amino, mono- ou di-alkylamino contenant 1 à 6 atomes de carbone par groupe alkyle,

$R^5$ représente l'hydrogène, un groupe alkyle en C1-C12, cycloalkyle en C3-C8,

$R^7$ représente un groupe phényle ou phényl-alkyle en C1-C3 dans lesquels le noyau phényle peut lui-même porter 1 à 3 des substituants suivants : des halogènes, des groupes alkyle en C1-C4, alcoxy en C1-C4, halogénoalkyle en C1-C4, amino ou mono- ou di-alkylamino contenant 1 à 4 atomes de carbone par groupe alkyle ;

un groupe hydroxy, un halogène, un groupe mercapto, alcoxy en C1-C4, alkylthio en C1-C4, amino, mono- ou di-alkylamino contenant 1 à 6 atomes de carbone par groupe alkyle,

un groupe phénoxy ou phénylthio dans lesquels le noyau phényle peut lui-même porter 1 à 3 des substituants suivants : les halogènes; les groupes alkyle en C1-C4, alcoxy en C1-C4, halogénoalkyle en C1-C4, amino ou mono- ou di-alkylamino contenant 1 à 4 atomes de carbone par groupe alkyle ;

un groupe halogéno-alkyle en C1-C3, (alcoxy en C1-C4)-alkyle en C1-C3, (alkyle en C1-C4)-thioalkyle en C1-C3, aminoalkyle en C1-C3, acyloxy en C1-C4 qui peut porter 1 à 3 groupes alkyle en C1-C4 et/ou un groupe acyle en C1-C4 ; hydrazino, cyano et thiocyanato,

$R^6$ représente l'hydrogène, un groupe alkyle en C1-C12, cycloalkyle en C3-C8, phényle ou phényl-alkyle en C1-C3 dans lesquels le noyau phényle peut lui-même porter 1 à 3 des substituants suivants : les halogènes, les groupes alkyle en C1-C4, alcoxy en C1-C4, halogénoalkyle en C1-C4, amino ou mono- ou di-alkylamino contenant 1 à 4 atomes de carbone par groupe alkyle;

un halogène, un groupe halogénoalkyle en C1-C3, (alcoxy en C1-C4)-alkyle en C1-C3, (alkylthio en C1-C4)-alkyle en C1-C3, aminoalkyle en C1-C3, carboxyle, (alcoxy en C1-C4)-carbonyle,

sous réserve que $R^1$ ne peut représenter un groupe amino lorsque $R^5$ et $R^6$ représentent l'hydrogène et $R^7$ l'hydrogène ou un groupe méthyle,

et leurs sels tolérés par les végétaux.

2. Procédé de préparation des composés I de la revendication 1, caractérisé en ce que l'on fait réagir la 2,6-diaminopyridine de formule II

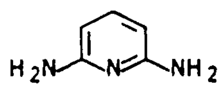

II

avec un dérivé $\beta$-dicarbonylé de formule générale III

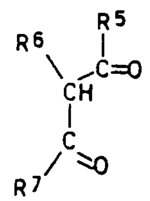

III

après quoi le cas échéant on prépare un dérivé ou bien on introduit des substituants sur les radicaux $R^1$ et/ou $R^5$ et/ou $R^6$ et/ou $R^7$ de manière connue en soi.

**3.** Produits herbicides contenant au moins une 1,8-naphtyridine substituée de formule générale I de la revendication 1 ou l'un de ses sels tolérés par les végétaux et au moins une substance active herbicide choisie dans le groupe suivant :

a) les dérivés d'acides 2-(4-hétéroaryloxy)- ou 2-(4-aryloxy)-phénoxyacétiques de formule IV

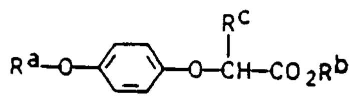

IV

dans laquelle les symboles ont les significations suivantes :
$R^a$ représente un noyau phényle, un noyau pyridyle, un radical benzoxazyle, un radical benzothiazyle ou un radical benzopyrazinyle, ces système cycliques aromatiques pouvant porter jusqu'à deux des substituants suivants : les halogènes, les groupes nitro, alkyle en C1-C4, halogénoalkyle en C1-C4 et/ou halogéno-alcoxy en C1-C4,
$R^b$ représente l'hydrogène, un groupe alkyle en C1-C4 ou l'équivalent d'un cation toléré par les végétaux et
$R^c$ représente l'hydrogène ou un groupe méthyle,
et
b) les dérivés de cyclohexénone de formule

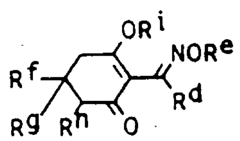

V

dans laquelle les symboles ont les significations suivantes :
$R^d$ représente un groupe alkyle en C1-C4 ;
$R^e$ représente un groupe alkyle en C1-C4, alcényle en C3-C4, alcynyle en C3-C4, halogénoalkylène en C3-C4 ou un groupe thényle qui peut être substitué par un atome d'halogène,
$R^f$ représente un groupe alkyle en C1-C4 qui peut être substitué par un ou deux groupes alkylthio en C1-C4 ou alcoxy en C1-C4 ;
un système cyclique saturé ou mono-insaturé à 5 ou 6 chaînons qui, en plus des chaînons carbonés, peut contenir un atome d'oxygène ou de soufre ou un groupe sulfoxyde ou sulfone, ce cycle pouvant porter jusqu'à 3 des substituants suivants : les groupes hydroxy, des halogènes, les groupes alkyle en C1-C4, halogénoalkyle en C1-C4, alcoxy en C1-C4 et/ou alkylthio en C1-C4;
un hétérocycle saturé ou mono-insaturé à 10 chaînons qui contient deux atomes d'oxygène ou de soufre et qui peut être substitué par 1 à 3 groupes alkyle en C1-C4 et/ou méthoxy ; un groupe phényle, pyridyle, thiazolyle, pyrazolyle, pyrrolyle ou isoxazolyle, ces groupes pouvant porter jusqu'à trois des substituants suivants : les groupes alkyle en C1-C4, halogénoalkyle en C1-C4, alcoxy en C1-C4, alkylthio en C1-C4, alcényloxy en C3-C6, alcynyloxy en C3-C6, (alcoxy en C1-C4)-alkyle en C1-C3, di-(alcoxy en C1-C4)-alkyle en C1-C3, formyle, les halogènes et/ou les groupes benzoylamino,

$R^g$ représente l'hydrogène, un groupe hydroxy ou bien encore, lorsque $R^f$ représente un groupe alkyle en C1-C6, un groupe alkyle en C1-C6 ;
$R^h$ représente l'hydrogène, un groupe cyano, un atome d'halogène ou un groupe (alcoxy en C1-C4)-carbonyle ou un groupe (alkyle en C1-C4)-cétoxime et
$R^i$ représente l'hydrogène ou l'équivalent d'un cation non polluant.

4. Produit herbicide selon la revendication 3, contenant une 1,8-naphtyridine substituée I selon la revendication 3 et un produit herbicide a) ou b), à des proportions relatives de 10 : 1 à 0,01 : 1 parties en poids.

5. Procédé pour combattre sélectivement les croissances de végétaux indésirables, caractérisé en ce que l'on applique avant, avec ou après les semis de la plante cultivée, avant ou durant sortie de terre de la plante cultivée, simultanément ou successivement dans un ordre quelconque, une 1,8-naphtyridine substituée de formule I selon la revendication 3 et un dérivé d'acide 2-(4-hétéroaryloxy)- ou 2-(4-aryloxy)-phénoxyacétique de formule IV ou un dérivé de cyclohexénone de formule V selon la revendication 3.

6. Procédé pour éviter les dommages causés aux végétaux cultivés par les dérivés d'acides 2-(4-hétéroaryloxy)- ou 2-(4-aryloxy)-phénoxyacétiques herbicides de formule IV ou les dérivés de cyclohexénone herbicides de formule V selon la revendication 3, caractérisé en ce que l'on traite les semences de la plante cultivée par une quantité antagoniste efficace d'une 1,8-naphtyridine substituée de formule I selon la revendication 3.

7. Procédé pour combattre sélectivement les croissances de végétaux indésirables, caractérisé en ce que l'on traite le feuillage des végétaux cultivés et des végétaux indésirables, en post-levée, simultanément ou successivement, par une 1,8-naphtyridine substituée de formule I selon la revendication 3 et par un dérivé d'acide 2-(4-hétéroaryloxy)- ou 2-(4-aryloxy)-phénoxyacétique de formule IV ou dérivé de cyclohexénone de formule V selon la revendication 3.

8. Procédé selon les revendications 5 à 7, caractérisé en ce que les végétaux cultivés sont l'orge, le blé, le maïs, le sorgho cultivé et le riz.

**Revendications pour l'Etat contractant suivant : ES**

1. Procédé de préparation des 1,8-naphtyridines substituées de formule générale I

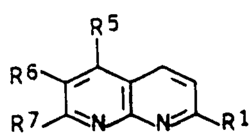

I,

dans laquelle les symboles ont les significations suivantes :
$R^1$ représente un groupe mercapto, benzylthio, sulfonyle, amino ou $NR^2R^3$ dans lequel $R^2$ représente un groupe alkyle en C1-C12, alcényle en C3-C6, cycloalkyle en C3-C8, phényle, phényl-alkyle en C1-C3, un groupe phényle ou phényl-alkyle en C1-C3 portant un à trois substituants choisis parmi les halogènes, les groupes alkyle en C1-C4, alcoxy en C1-C4, amino, mono- ou di-alkylamino contenant 1 à 6 atomes de carbone par groupe alkyle ; ou bien un groupe

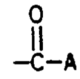

,

dans lequel A représente un groupe alkyle en C1-C8 phényle, un groupe phényle portant 1 à 3 substituants choisis parmi les halogènes, les groupes alkyle en C1-C4, alcoxy en C1-C4, amino, mono- ou di-alkylamino contenant 1 à 6 atomes de carbone par groupe alkyle, un groupe amino, alkyl- ou dialkyl-amino contenant 1 à 6 atomes de carbone par groupe alkyle, phénylamino, morpholino ou

pipéridyle, et
$R^3$ représente l'hydrogène, un groupe alkyle en C1-C12, cycloalkyle en C3-C8, ou bien
$R^2$ et $R^3$ représentent ensemble un hétérocycle saturé ou insaturé à 5 ou 6 chaînons qui, en plus de l'atome d'azote auquel ils sont réliés, peut contenir un autre hétéroatome tel que l'oxygène ou l'azote ;
un groupe hydroxy, marcapto, un halogène ou bien
un groupe $XR^4$ dans lequel X représente l'oxygène ou le soufre et R4 un groupe alkyle en C1-C12, (alcoxy en C1-C4)-carbonyl-alkyle en C1-C4, cycloalkyle en C5-C8, phényle ou phényl-alkyle en C1-C3 dont les noyaux aromatiques peuvent porter 1 à 3 des substituants suivants :
des halogènes, des groupes alkyle en C1-C4, alcoxy en C1-C4, amino, mono- ou di-alkylamino contenant 1 à 6 atomes de carbone par groupe alkyle,
$R^5$ représente l'hydrogène, un groupe alkyle en C1-C12, cycloalkyle en C3-C8,
$R^7$ représente un groupe phényle ou phényl-alkyle en C1-C3 dans lesquels le noyau phényle peut lui-même porter 1 à 3 des substituants suivants : des halogènes, des groupes alkyle en C1-C4, alcoxy en C1-C4, halogénoalkyle en C1-C4, amino ou mono- ou di-alkylamino contenant 1 à 4 atomes de carbone par groupe alkyle ;
un groupe hydroxy, un halogène, un groupe mercapto, alcoxy en C1-C4, alkylthio en C1-C4, amino, mono- ou di-alkylamino contenant 1 à 6 atomes de carbone par groupe alkyle,
un groupe phénoxy ou phénylthio dans lesquels le noyau phényle peut lui-même porter 1 à 3 des substituants suivants : les halogènes; les groupes alkyle en C1-C4, alcoxy en C1-C4, halogénoalkyle en C1-C4, amino ou mono- ou di-alkylamino contenant 1 à 4 atomes de carbone par groupe alkyle ;
un groupe halogéno-alkyle en C1-C3, (alcoxy en C1-C4)-alkyle en C1-C3, (alkyle en C1-C4)-thioalkyle en C1-C3, aminoalkyle en C1-C3, acyloxy en C1-C4 qui peut porter 1 à 3 groupes alkyle en C1-C4 et/ou un groupe acyle en C1-C4 ; hydrazino, cyano et thiocyanato,
$R^6$ représente l'hydrogène, un groupe alkyle en C1-C12, cycloalkyle en C3-C8, phényle ou phényl-alkyle en C1-C3 dans lesquels le noyau phényle peut lui-même porter 1 à 3 des substituants suivants : les halogènes, les groupes alkyle en C1-C4, alcoxy en C1-C4, halogénoalkyle en C1-C4, amino ou mono- ou di-alkylamino contenant 1 à 4 atomes de carbone par groupe alkyle ;
un halogène, un groupe halogénoalkyle en C1-C3, (alcoxy en C1-C4)-alkyle en C1-C3, (alkylthio en C1-C4)-alkyle en C1-C3, aminoalkyle en C1-C3, carboxyle, (alcoxy en C1-C4)-carbonyle,
sous réserve que $R^1$ ne peut représenter un groupe amino lorsque $R^5$ et $R^6$ représentent l'hydrogène et $R^7$ l'hydrogène ou un groupe méthyle,
et leurs sels tolérés par les végétaux,
caractérisé en ce que l'on fait réagir 2,6-diaminopyridine de formule II

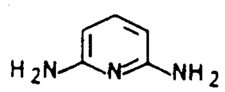

II

avec un dérivé $\beta$-dicarbonylé de formule générale III

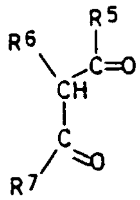

III

après quoi le cas échéant on prépare un dérivé ou bien on introduit des substituants sur les radicaux $R^1$ et/ou $R^5$ et/ou $R^6$ et/ou $R^7$ de manière connue en soi.

**2.** Produits herbicides contenant au moins une 1,8-naphtyridine substituée de formule générale I de la revendication 1 ou l'un de ses sels tolérés par les végétaux et au moins une substance active herbicide choisie dans le groupe suivant :

a) les dérivés d'acides 2-(4-hétéroaryloxy)- ou 2-(4-aryloxy)-phénoxyacétiques de formule IV

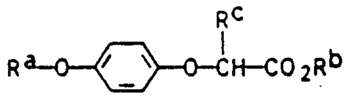

IV

dans laquelle les symboles ont les significations suivantes :

$R^a$ représente un noyau phényle, un noyau pyridyle, un radical benzoxazyle, un radical benzothiazyle ou un radical benzopyrazinyle, ces système cycliques aromatiques pouvant porter jusqu'à deux des substituants suivants : les halogènes, les groupes nitro, alkyle en C1-C4, halogénoalkyle en C1-C4 et/ou halogéno-alcoxy en C1-C4,

$R^b$ représente l'hydrogène, un groupe alkyle en C1-C4 ou l'équivalent d'un cation toléré par les végétaux et

$R^c$ représente l'hydrogène ou un groupe méthyle,

et

b) les dérivés de cyclohexénone de formule

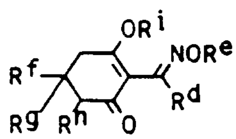

V

dans laquelle les symboles ont les significations suivantes :

$R^d$ représente un groupe alkyle en C1-C4 ;

$R^e$ représente un groupe alkyle en C1-C4, alcényle en C3-C4, alcynyle en C3-C4, halogénoalkylène en C3-C4 ou un groupe thényle qui peut être substitué par un atome d'halogène,

$R^f$ représente un groupe alkyle en C1-C4 qui peut être substitué par un ou deux groupes alkylthio en C1-C4 ou alcoxy en C1-C4 ;

un système cyclique saturé ou mono-insaturé à 5 ou 6 chaînons qui, en plus des chaînons carbonés, peut contenir un atome d'oxygène ou de soufre ou un groupe sulfoxyde ou sulfone, ce cycle pouvant porter jusqu'à 3 des substituants suivants : les groupes hydroxy, des halogènes, les groupes alkyle en C1-C4, halogénoalkyle en C1-C4, alcoxy en C1-C4 et/ou alkylthio en C1-C4 ;

un hétérocycle saturé ou mono-insaturé à 10 chaînons qui contient deux atomes d'oxygène ou de soufre et qui peut être substitué par 1 à 3 groupes alkyle en C1-C4 et/ou méthoxy ; un groupe phényle, pyridyle, thiazolyle, pyrazolyle, pyrrolyle ou isoxazolyle, ces groupes pouvant porter jusqu'à trois des substituants suivants : les groupes alkyle en C1-C4, halogénoalkyle en C1-C4, alcoxy en C1-C4, alkylthio en C1-C4, alcényloxy en C3-C6, alcynyloxy en C3-C6, (alcoxy en C1-C4)-alkyle en C1-C3, di-(alcoxy en C1-C4)-alkyle en C1-C3, formyle, les halogènes et/ou les groupes benzoylamino,

$R^g$ représente l'hydrogène, un groupe hydroxy ou bien encore, lorsque $R^f$ représente un groupe alkyle en C1-C6, un groupe alkyle en C1-C6 ;

$R^h$ représente l'hydrogène, un groupe cyano, un atome d'halogène ou un groupe (alcoxy en C1-C4)-carbonyle ou un groupe (alkyle en C1-C4)-cétoxime et

$R^i$ représente l'hydrogène ou l'équivalent d'un cation non polluant.

**3.** Produit herbicide selon la revendication 2, contenant une 1,8-naphtyridine substituée selon la revendication 2 et un produit herbicide a) ou b) à des proportions relatives de 10 : 1 à 0,01 : 1 parties en poids.

**4.** Procédé pour combattre sélectivement les croissances de végétaux indésirables, caractérisé en ce que l'on applique avant, avec ou après les semis de la plante cultivée, avant ou durant la sortie de terre de la plante cultivée, simultanément ou successivement dans un ordre quelconque, une 1,8-naphtyridine substituée de formule I selon la revendication 2 et un dérivé d'acide 2-(4-hétéroaryloxy)- ou 2-(aryloxy)-phénoxyacétique de formule IV ou un dérivé de cyclohexénone de formule V selon la revendication 2.

5. Procédé pour éviter les dommages causés aux végétaux cultivés par les dérivés d'acides 2-(4-hétéroaryloxy)- ou 2-(4-aryloxy)-phénoxyacétiques herbicides de formule IV ou les dérivés de cyclohexénone herbicides de formule V selon la revendication 2, caractérisé en ce que l'on traite les semences de la plante cultivée par une quantité antagoniste efficace d'une 1,8-naphtyridine substituée de formule I selon la revendication 2.

6. Procédé pour combattre sélectivement les croissances de végétaux indésirables, caractérisé en ce que l'on traite le feuillage de la plante cultivée et des végétaux indésirables en post-levée par une 1,8-naphtyridine substituée de formule I selon la revendication 2 et par un dérivé d'acide 2-(4-hétéroaryloxy) ou 2(4-aryloxy)-phénoxyacétique de formule IV ou un dérivé de cyclohexénone de formule V selon la revendication 2, simultanément ou successivement.

7. Procédé selon les revendications 4 à 6, caractérisé en ce que les plantes cultivées sont l'orge, le blé, le maïs, le sorgho cultivé et le riz.